(19) Europäisches Patentamt
European Patent Office
Office européen des brevets

(11) Veröffentlichungsnummer: **0 074 929**
**A1**

(12) # EUROPÄISCHE PATENTANMELDUNG

(21) Anmeldenummer: 82810381.2

(22) Anmeldetag: 13.09.82

(51) Int. Cl.³: C 07 D 487/04
C 07 D 471/14, A 61 K 31/495
//(C07D487/04, 249/00, 241/00),
(C07D471/14, 249/00, 241/00,
221/00)

(30) Priorität: 16.09.81 CH 5975/81

(43) Veröffentlichungstag der Anmeldung:
23.03.83 Patentblatt 83/12

(84) Benannte Vertragsstaaten:
AT BE CH DE FR IT LI LU NL SE

(71) Anmelder: CIBA-GEIGY AG
Patentabteilung Postfach
CH-4002 Basel(CH)

(72) Erfinder: Heckendorn, Roland, Dr.
Blumenweg 20
CH-4144 Arlesheim(CH)

(54) **Neue polycyclische Polyazaheterocyclen, Verfahren zu deren Herstellung und diese enthaltende pharmazeutische Präparate.**

(57) Die Erfindung betrifft neue polycylische Polyazaheterocyclen mit psychopharmakologischen Eigenschaften, insbesondere antidepressiver und anxiolytischer Wirksamkeit, entsprechend der allgemeinen Formel I

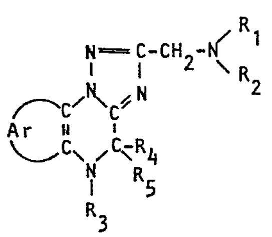

(1)

in welcher $R_1$ und $R_2$ unabhängig voneinander Wasserstoff, Niederalkyl oder Hydroxyniederalkyl, oder zusammen Niederalkylen oder Aethylenoxyäthylen, Aeithylenazaäthylen oder N-Niederalkyl- oder N-(2-Hydroxyniederalkyl)-äthylenazaäthylen, $R_3$ Wasserstoff oder einen niederen aliphatischen oder gesättigten, niederen cycloaliphatischen Kohlenwasserstoffrest oder unsubstituiertes oder substituiertes Phenyl, $R_4$ und $R_5$ Wasserstoff oder Niederalkyl und Ar einen unsubstituierten oder substituierten Benzo- oder Pyridorest bedeutet, und die Säureadditionssalze der Verbindungen der allgemeinen Formel I, insbesondere die pharmazeutisch annehmbaren Säureadditionssalze von Verbindungen der allgemeinen Formel I, sowie Verfahren zur Herstellung dieser neuen Stoffe und solche enthaltende pharmazeutische Präparate.

EP 0 074 929 A1

Croydon Printing Company Ltd.

CIBA-GEIGY AG 4-13554/=

Basel (Schweiz)

# Neue polycyclische Polyazaheterocyclen, Verfahren zu deren Herstellung und diese enthaltende pharmazeutische Präparate.

Die Erfindung betrifft neue polycyclische Polyazaheterocyclen und deren Säureadditionssalze, Verfahren zu deren Herstellung und diese enthaltende pharmazeutische Präparate.

Die erfindungsgemässen neuen Verbindungen entsprechen der allgemeinen Formel I

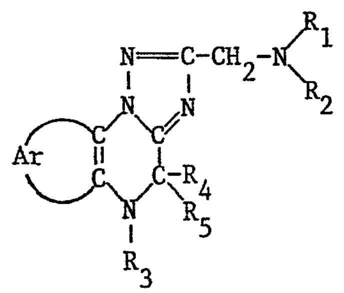

(I)

in welcher $R_1$ und $R_2$ unabhängig voneinander Wasserstoff, Niederalkyl oder Hydroxyniederalkyl, oder zusammen Niederalkylen oder Aethylenoxyäthylen, Aethylenazaäthylen oder N-Niederalkyl- oder N-(2-Hydroxyniederalkyl)-äthylenazaäthylen, $R_3$ Wasserstoff oder einen niederen aliphatischen oder gesättigten, niederen cycloaliphatischen Kohlenwasserstoffrest oder unsubstituiertes oder substituiertes Phenyl, $R_4$ und $R_5$ Wasserstoff oder Niederalkyl und Ar einen unsubstituierten oder substituierten Benzo- oder Pyridorest bedeutet. Unter einem Benzo- oder Pyridorest wird ein zweiwertiger Rest verstanden, der zusammen mit den beiden anliegenden Kohlenstoffatomen einen Benzol- bzw. Pyridinring bildet. Ebenfalls Gegenstand der Erfindung sind die Säureadditionssalze der Verbindungen der allgemeinen Formel I, insbesondere die pharmazeutisch annehmbaren Säureadditionssalze von Verbindungen der allgemeinen Formel I.

Wenn nichts anderes vermerkt ist, werden vor- und nachstehend unter niederen Resten solche verstanden, die höchstens 7 und vorzugsweise höchstens 4 Kohlenstoffatome enthalten. Infolge der engen Beziehung zwischen den Verbindungen der allgemeinen Formel I in Form von freien Basen und in Form von Säureadditionssalzen sind nachfolgend unter den Basen oder ihren Säureadditionssalzen sinn- und zweckmässig gegebenenfalls auch die entsprechenden Säureadditionssalze bzw. freien Basen zu verstehen.

Niederalkyl $R_1$ und/oder $R_2$ ist z.B. Aethyl, Propyl, Isopropyl, Butyl oder Isobutyl und vor allem Methyl. In Hydroxyniederalkyl $R_1$ und/oder $R_2$ befindet sich das Hydroxy in höherer als der 1-Stellung, entsprechend sind solche Reste z.B. 2- oder 3-Hydroxypropyl, 2-, 3- oder 4-Hydroxybutyl und vor allem 2-Hydroxyäthyl. Als Niederalkylen bedeuten $R_1$ und $R_2$ zusammen z.B. Aethylen, Trimethylen, Hexamethylen und vor allem Tetramethylen oder Pentamethylen, oder entsprechend verzweigte, d.h. niederalkylierte, insbesondere methylierte Reste, wie z.B. 1,4-Dimethyltetramethylen oder 3,3-Dimethylpentamethylen. Das Niederalkyl im Aethylenazaäthylen ist z.B. Aethyl, Propyl, Isopropyl und vor allem Methyl, und das 2-Hydroxyniederalkyl z.B. 2-Hydroxypropyl und vor allem 2-Hydroxyäthyl.

Als niederer aliphatischer Kohlenwasserstoffrest ist $R_3$ Niederalkyl, Niederalkenyl oder Niederalkinyl mit höchstens je 7 und vorzugsweise höchstens je 4 Kohlenstoffatomen. Als Niederalkyl ist $R_3$ z.B. Methyl, Aethyl, Propyl, Isopropyl, Butyl, Isobutyl, sek.-Butyl, Pentyl, Isopentyl, Neopentyl, 1-Methylbutyl, 1-Aethylpropyl, Hexyl, Isohexyl, 1-Methylpentyl, Heptyl, Isoheptyl, 1-Methylhexyl oder 1-Propylbutyl, als Niederalkenyl insbesondere solches mit 3 bis 4 Kohlenstoffatomen, z.B. Allyl, 1-Methylallyl, 2-Methylallyl, 2-Butenyl oder 3-Butenyl, und als Niederalkinyl z.B. 2-Propinyl oder 2-Butinyl. Als gesättigter, niederer cycloaliphatischer Kohlenwasserstoffrest ist $R_3$ insbesondere Cycloalkyl, Niederalkylcycloalkyl oder Cycloalkylnie-

deralkyl mit jeweils höchstens 7 Kohlenstoffatomen, wie z.B. Cyclopentyl, Cyclohexyl, Cycloheptyl bzw. 2- oder 4-Methylcyclohexyl, bzw. Cyclopropylmethyl, Cyclopentylmethyl, 2-Cyclopentyläthyl oder Cyclohexylmethyl.

Substituiertes Phenyl $R_3$ ist z.B. durch Halogen bis Atomnummer 35, Niederalkyl, Niederalkoxy, Niederalkylthio, Trifluormethyl, Cyano und/oder Nitro ein- oder mehrfach substituiertes Phenyl.

In einem Pyridorest Ar kann sich das Ringstickstoffatom in jeder der 4 möglichen Stellungen befinden, insbesondere steht es in der 4-Stellung und vor allem in der 2-Stellung zum Ringkohlenstoffatom, das mit dem durch $R_3$ substituierten Stickstoffatom des Pyrazinringes verbunden ist. Als Pyridorest ist somit Ar, bezogen auf den zentralen Pyrazinring,insbesondere der Pyrido[4,3-e]- und vor allem der Pyrido-[2,3-e]-rest. Substituenten eines Benzo- oder Pyridorestes Ar sind z.B. Halogen bis Atomnummer 35, Niederalkyl, Niederalkoxy, Niederalkylthio, Trifluormethyl, Cyano oder Nitro.

Als Substituent von Phenyl $R_3$ und/oder als Substituent von Ar vorliegendes Halogen ist Fluor, Brom oder vor allem Chlor, Niederalkyl z.B. Aethyl, Propyl, Isopropyl, Butyl oder tert.-Butyl und insbesondere Methyl, Niederalkoxy z.B. Aethoxy, Propoxy, Isopropoxy, Butoxy oder Isobutoxy, und insbesondere Methoxy, und Niederalkylthio z.B. Aethylthio, Propylthio, Isopropylthio, Butylthio und insbesondere Methylthio. Niederalkyl $R_4$ ist z.B. Isopropyl oder vorzugsweise primäres Niederalkyl, wie Aethyl, Propyl, Isobutyl und vor allem Methyl und Niederalkyl, $R_5$ vorzugsweise eine der vorgenannten primären Niederalkyle, vor allem Methyl neben Methyl $R_4$.

Als Säureadditionssalze von Verbindungen der Formel I kommen insbesondere pharmazeutisch annehmbare Säureadditionssalze in Betracht. Solche können gegebenenfalls wie die freien Verbindungen der allgemeinen Formel I als Wirkstoffe für pharmazeutische Präparate verwendet

werden. Als Beispiele seien die Additionssalze mit der Chlorwasserstoffsäure, Bromwasserstoffsäure, Schwefelsäure, Phosphorsäure, Methansulfonsäure, Aethansulfonsäure, 2-Hydroxyäthansulfonsäure, Essigsäure, Milchsäure, Bernsteinsäure, Fumarsäure, Maleinsäure, Aepfelsäure, Weinsäure, Citronensäure, Benzoesäure, Salicylsäure, Phenylessigsäure, Mandelsäure und Embonsäure genannt.

Die Verbindungen der allgemeinen Formel I und ihre Säureadditionssalze besitzen wertvolle psychopharmakologische Eigenschaften. Insbesondere wirken sie antagonistisch gegenüber der durch Reserpin induzierten Hypothermie, z.B. bei intraperitonealer oder peroraler Applikation an der Ratte, [vgl. Benz und Waser, Arzneimittelforsch. 21 (5) 654 (1971)] im Dosenbereich von 10 bis 100 mg/kg, und gegenüber der durch Tetrabenazin induzierten Ptosis bei intraperitonealer oder peroraler Applikation an der Ratte [vgl. Rubin et al., J. Pharmacol. exptl. Ther. 120, 125 (1957)] im Dosenbereich von 3 bis 100 mg/kg. Beispielsweise beträgt die $ED_{50}$ von 4,5-Dihydro-2-[(dimethylamino)-methyl]-5-phenyl-1,2,4-triazolo[1,5-a]chinoxalin-hydrochlorid im zweitgenannten Test 3-10 mg/kg per os und 10 mg/kg i.p.. Im Sozialkonflikt-Test an der Ratte zeigen die Verbindungen der allgemeinen Formel I, wie die vorgenannte, anxiolytische Wirkung nach peroraler Verabreichung von Dosen ab 1 mg/kg. Im Gegensatz zur eher negativ inotropen und positiv chronotropen Wirkung mancher bekannter Antidepressiva, wie Imipramin, zeigen die Verbindungen der allgemeinen Formel I, wie die vorgenannte, am isolierten Meerschweinchenvorhof eine schwach positiv inotrope und schwach negativ chronotrope Wirkung. Die Toxizität der Verbindungen der allgemeinen Formel I ist im Vergleich zu den erwünschten pharmakologischen Wirkungen niedrig. Diese Eigenschaften kennzeichnen die Verbindungen der allgemeinen Formel I als Antidepressiva und Anxiolytica, die zur Behandlung von Depressionen Verwendung finden können.

Die Erfindung betrifft insbesondere Verbindungen der allgemeinen Formel I, in denen $R_1$ und $R_2$ die unter der Formel I angegebene Bedeu-

tung haben, $R_3$ einen niederen aliphatischen oder einen gesättigten niederen cycloaliphatischen Kohlenwasserstoffrest oder unsubstituiertes oder durch Halogen bis Atomnummer 35, Niederalkyl, Niederalkoxy, Niederalkylthio, Trifluormethyl, Cyano oder Nitro substituiertes Phenyl und Ar einen unsubstituierten oder durch Substituenten der vorgenannten Art substituierten Benzo- oder Pyrido[2,3-e]-rest bedeutet und $R_4$ und $R_5$ die unter Formel I angegebene Bedeutung haben, und deren Säureadditionssalze, insbesondere die pharmazeutisch annehmbaren Säureadditionssalze.

Die Erfindung betrifft ganz besonders Verbindungen der allgemeinen Formel I, in denen $R_1$ Niederalkyl, $R_2$ Wasserstoff oder Niederalkyl, oder $R_1$ und $R_2$ zusammen Niederalkylen, $R_3$ unsubstituiertes oder durch Halogen bis Atomnummer 35 substituiertes Phenyl oder niederes Cycloalkyl und Ar einen unsubstituierten oder durch Halogen bis Atomnummer 35 substituierten Benzo- oder Pyrido[2,3-e]rest bedeutet, und $R_4$ und $R_5$ die unter der Formel I angegebene Bedeutung haben, und deren Säureadditionssalze, insbesondere die pharmazeutisch annehmbaren Säureadditionssalze.

Die Erfindung betrifft vor allem Verbindungen der allgemeinen Formel I, in denen $R_1$ Niederalkyl, insbesondere Methyl, $R_2$ Wasserstoff oder Niederalkyl, insbesondere Methyl, oder $R_1$ und $R_2$ zusammen Niederalkylen, insbesondere Tetramethylen, $R_3$ unsubstituiertes oder durch Halogen bis Atomnummer 35, insbesondere Chlor, substituiertes Phenyl, und Ar einen unsubstituierten oder durch Halogen bis Atomnummer 35, insbesondere Chlor substituierten Benzorest oder den Pyrido[2,3-e]-rest bedeutet, $R_4$ die unter Formel I angegebene Bedeutung hat und $R_5$ Wasserstoff oder Methyl bedeutet, und deren Säureadditionssalze, insbesondere die pharmazeutisch annehmbaren Säureadditionssalze.

Die Erfindung betrifft in erster Linie Verbindungen der allgemeinen Formel I, in welcher $R_1$ Methyl, $R_2$ Wasserstoff oder Methyl, $R_3$ Phenyl, $R_4$ Wasserstoff oder Methyl, $R_5$ Methyl oder vor allem Wasserstoff, und Ar den Benzo- oder Pyrido[2,3-e]-rest bedeutet, und deren Säureadditionssalze, insbesondere die pharmazeutisch annehmbaren Säureadditionssalze.

Die neuen Verbindungen werden in an sich bekannter Weise hergestellt, indem man z.B.

a) einen reaktionsfähigen Ester einer Verbindung der allgemeinen Formel II

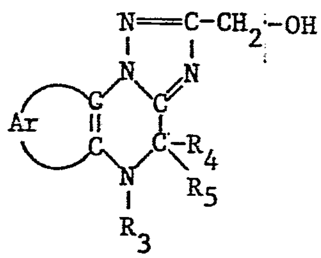 (II),

in welcher

$R_3$, $R_4$, $R_5$ und Ar die unter der Formel I angegebene Bedeutung haben, mit einer Verbindung der allgemeinen Formel III

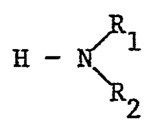 (III),

in welcher $R_1$ und $R_2$ die unter der Formel I angegebene Bedeutung haben, umsetzt, oder

b) in einer Verbindung der allgemeinen Formel IV

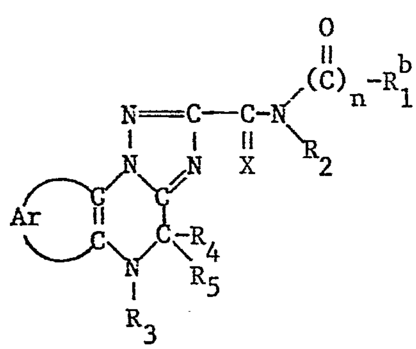 (IV),

in welcher n Null oder 1 und X Sauerstoff bedeutet, oder, falls n 1 bedeutet, auch zwei Wasserstoffatome bedeuten kann, $R_1^b$ allein und zusammen mit $R_2$ die Bedeutung von $R_1$ hat oder, falls n 1 bedeutet, auch Niederalkoxy bedeuten kann und $R_2$, $R_3$, $R_4$, $R_5$ und Ar die unter der Formel I angegebene Bedeutung haben, mittels eines Hydrid-Reduktionsmittels

die Carbonylgruppe(n) bzw. die gegebenenfalls vorhandene Niederalkoxycarbonylgruppe reduziert, oder

c) in eine Verbindung der allgemeinen Formel I, in welcher $R_3$ Wasserstoff bedeutet und $R_1$, $R_2$, $R_4$, $R_5$ und Ar die unter der Formel I angegebene Bedeutung haben, einen von Wasserstoff verschiedenen Rest $R_3$ einführt, an dessen Bindungskohlenstoffatom sich noch mindestens ein Wasserstoffatom befindet, oder

d) in eine Verbindung der allgemeinen Formel I, in welcher ein oder beide Symbole $R_1$ und $R_2$ Wasserstoff bedeuten, und $R_3$, $R_4$, $R_5$ und Ar die unter der Formel I angegebene Bedeutung haben, einen oder zwei von Wasserstoff verschiedene Reste $R_1$ und/oder $R_2$ einführt,

und gewünschtenfalls eine erhaltene Verbindung der allgemeinen Formel I in ein Additionssalz mit einer anorganischen oder organischen Säure überführt oder aus einem erhaltenen Säureadditionssalz die Base freisetzt und letztere gewünschtenfalls wiederum in ein Säureadditionssalz überführt.

Für die Umsetzungen gemäss a) eignen sich als reaktionsfähige Ester von Verbindungen der allgemeinen Formel II beispielsweise Ester organischer Sulfonsäuren, wie niedere Alkansulfonsäureester oder Arensulfonsäureester, z.B. Methansulfonsäureester bzw. Benzolsulfonsäure- oder p-Toluolsulfonsäureester, oder Halogenide, insbesondere Bromide, Chloride oder Jodide. Die Umsetzungen werden beispielsweise in einem inerten organischen Lösungsmittel bei Temperaturen von etwa 0°C bis etwa 100°C bzw. Siedetemperatur des verwendeten Lösungsmittels, falls diese tiefer als 100°C liegt, in Gegenwart eines Ueberschusses an der umzusetzenden Verbindung der allgemeinen Formel III oder einer tertiären organischen oder einer anorganischen Base, z.B. Triäthylamin, Aethyl-diisopropylamin oder Pyridin, bzw. einem Alkalimetallcarbonat oder -bicarbonat, wie Kaliumcarbonat oder Natriumbicarbonat, als säurebindendem Mittel durchgeführt.

Die Reduktion von Verbindungen der allgemeinen Formel IV gemäss b) kann beispielsweise mittels eines Niederalkylaluminiumhydrids, wie Diisobutylaluminiumhydrid, z.B. in einem aromatischen Kohlenwasserstoff, wie Toluol, bei Temperaturen zwischen etwa -10°C und Raumtemperatur, oder mittels Lithiumaluminiumhydrid oder Diboran in einem ätherartigen Lösungsmittel, wie Diäthyläther oder Tetrahydrofuran, z.B. bei Temperaturen zwischen Raumtemperatur und etwa 55°C bzw. Siedetemperatur des Lösungsmittels, falls diese unterhalb 55°C liegt, durchgeführt werden.

Die Einführung eines von Wasserstoff verschiedenen, am Bindungskohlenstoffatom noch mindestens ein Wasserstoffatom aufweisenden und somit weder aromatischen noch über ein quartäres Kohlenstoffatom gebundenen Restes $R_3$ in eine Verbindung der allgemeinen Formel I, in der $R_3$ Wasserstoff ist, gemäss c) erfolgt insbesondere durch Umsetzung einer der letztgenannten Verbindungen mit einem reaktionsfähigen Ester eines sekundären oder vorzugsweise primären Niederalkanols, Niederalkenols oder Niederalkinols, oder eines Cycloalkanols mit 5 bis 7 Kohlenstoffatomen oder eines vorzugsweise primären Cycloalkylniederalkanols mit 4 bis 7 Kohlenstoffatomen. Z.B. verwendet man als entsprechende reaktionsfähige Ester Halogenwasserstoffsäureester, wie Chloride oder insbesondere Bromide oder Jodide, oder Ester mit organischen Sulfonsäuren, wie Niederalkansulfonsäure- oder Arensulfonsäureester, z.B. Methansulfonsäureester bzw. Benzolsulfonsäure- oder p-Toluolsulfonsäureester, von Methanol, Aethanol, Propanol, Isopropanol, Butanol, Isobutanol, Allylalkohol, 2-Methylallylalkohol, 2-Propinol, Cyclopentanol, Cyclohexanol, Cycloheptanol, Cyclopropyl-, Cyclobutyl-, Cyclopentyl- oder Cyclohexylmethanol oder 2-Cyclopentyläthanol. Die Umsetzung kann in einem inerten organischen Lösungs- oder Verdünnungsmittel und beispielsweise bei Temperaturen zwischen etwa 0°C und 100°C, insbesondere zwischen Raumtemperatur und etwa 80°C bzw. Siedetemperatur des Lösungsmittels, wenn diese unterhalb 80°C liegt, in Gegenwart eines basischen Kondensationsmittels, z.B. von

einem Alkalimetall, Alkalimetall-amid oder -hydrid, oder einem Alkalimetallniederalkoxid, wie Natrium- oder Kalium-methoxid, -äthoxid oder tert.-butoxid, durchgeführt werden.

Einen primären Rest $R_3$ kann man z.B. auch durch eine zweistufige Reaktionsfolge einführen, indem man die Verbindung der allgemeinen Formel I zunächst zur entsprechenden Verbindung mit dem Acylrest einer Niederalkansäure oder Cycloalkylniederalkansäure mit höchstens 7 Kohlenstoffatomen in 5-Stellung acyliert und letztere Verbindung mittels eines Hydrid-Reduktionsmittels analog zum Verfahren b) reduziert.

Die Einführung von einem bzw. zwei von Wasserstoff verschiedenen Resten $R_1$ und/oder $R_2$ in eine Verbindung der allgemeinen Formel I, in welcher mindestens eines der Symbole $R_1$ und $R_2$ Wasserstoff bedeutet, kann beispielsweise durch Umsetzung einer entsprechenden Verbindung der allgemeinen Formel I mit der äquimolaren oder gegebenenfalls mindestens doppeltmolaren Menge eines reaktionsfähigen Esters, z.B. eines Halogenwasserstoffsäureesters, wie eines Chlorids oder insbesondere Bromids oder Jodids, oder eines Esters mit einer organischen Sulfonsäure, z.B. eines Niederalkansulfonsäure- oder Arensulfonsäureesters, wie des Methansulfonsäureesters bzw. des Benzolsulfonsäure oder p-Toluolsulfonsäureesters eines nicht-tertiären Niederalkanols oder eines entsprechenden Mono- oder Diesters eines Niederalkandiols, z.B. eines entsprechenden Esters von Methanol, Aethanol, Propanol, Isopropanol oder Butanol, eines entsprechenden Monoesters von 1,2-Aethandiol oder 1,2- oder 1,3-Propandiol oder eines entsprechenden Mono- oder Diesters von 1,4-Butandiol oder 1,5-Pentandiol, im wesentlichen analog zu den für Verfahren a) angegebenen Reaktionsbedingungen erfolgen.

Eine weitere Möglichkeit zur Einführung von Resten $R_1$ und/oder $R_2$, die von Wasserstoff verschieden sind, in hierfür geeignete Verbindungen der allgemeinen Formel I, besteht in der Umsetzung solcher

Verbindungen mit Oxoniederalkanen, wie z.B. Formaldehyd, Acetaldehyd, Aceton, 2-Butanon oder 3-Pentanon unter reduzierenden Bedingungen, z.B. in Gegenwart von Ameisensäure bei Temperaturen von etwa 60°C bis etwa 100°C oder in einem inerten organischen Lösungsmittel in Gegenwart von Wasserstoff und eines üblichen Hydrierungskatalysators, z.B. eines Edelmetallkatalysators, wie Palladium auf Kohle oder einem Erdalkalimetallcarbonat, oder Platinoxid, oder von Raney-Nickel, bei Raumtemperatur und Normaldruck oder mässig erhöhten Temperaturen und Drücken. Bei Verwendung von Aldheyden, insbesondere Formaldehyd, vor allem in Gegenwart von Ameisensäure, werden alle vorhandenen Wasserstoffatome durch Niederalkyl, z.B. Methyl, ersetzt, während man bei Verwendung von Ketonen in Gegenwart von Wasserstoff und Hydrierungskatalysatoren auch Verbindungen der allgemeinen Formel I mit sekundärem Niederalkyl als $R_1$ und Wasserstoff als $R_2$ erhalten kann.

Die Verbindungen der allgemeinen Formel II sind ebenfalls neue Stoffe. Zur Herstellung solcher Verbindungen, in denen Ar einen Benzorest und $R_5$ Wasserstoff bedeutet, während $R_3$ und $R_4$ die unter der Formel I angegebene Bedeutung haben, kann man z.B. von in der Aminogruppe gegebenenfalls entsprechend der Definition für $R_3$ und gegebenenfalls im Benzolring substituierten o-Nitroanilinen ausgehen. In deren Aminogruppe wird zunächst eine geeignete Schutzgruppe, wie eine Niederalkoxycarbonylgruppe, z.B. die Aethoxycarbonylgruppe durch aufeinanderfolgende Einwirkung von Natriumhydrid und Chlorameisensäureäthylester in Dimethylformamid, eingeführt und im erhaltenen Produkt die Nitrogruppe in üblicher Weise, z.B. mittels Wasserstoff in Gegenwart von Raney-Nickel, zur Aminogruppe reduziert. Das Reduktionsprodukt wird durch Lösung in einem Salzsäure-Essigsäure-Gemisch und Zufügen von Natriumnitritlösung in das entsprechende Diazoniumchlorid übergeführt und letzteres mit einem (2-Chlorniederalkanamido)-malonsäuredinieder-alkylester, wie dem Diäthylester, zur entsprechenden Azoverbindung, z.B. dem entsprechenden [2-(N-Alkoxycarbonyl-N-$R_3$-amino)-phenylazo]-(2-chlorniederalkanamido)-malonsäurediäthylester, gekuppelt. Durch

Behandeln des letzteren mit wässriger Natriumhydroxidlösung bei Raumtemperatur und Ansäuern, z.B. mit Salzsäure in der Kälte, entsteht unter Hydrolyse, Decarboxylierung und anschliessender Kondensation die entsprechende 1-[2-(N-Aethoxycarbonyl-N-$R_3$-amino)-phenyl]-5-(1-chlorniederalkyl)-1H-1,2,4-triazol-3-carbonsäure, die ihrerseits bei der Abspaltung der Aethoxycarbonylgruppe, z.B. mittels konz. Bromwasserstoffsäure, in der Wärme, z.B. bei etwa 90°C, zur entsprechenden 4-$R_4$-5-$R_3$-4,5-dihydro-[1,2,4]-triazolo[1,5-a]chinoxalin-2-carbonsäure cyclisiert. Diese kann im Benzolring, d.h. in einer der Stellungen 6, 7, 8 oder 9 Substituenten enthalten, sofern von einem entsprechend im Benzolring substituierten N-$R_3$-2-nitroanilin ausgegangen wurde. Die erhaltene Carbonsäure kann schliesslich direkt oder nach Veresterung, z.B. Ueberführung in den Methylester durch Kochen mit Methanol und Salzsäure, mittels eines Hydrid-Reduktionsmittels, z.B. Behandlung mit Natriumborhydrid in einem Gemisch von Methanol und Tetrahydrofuran, zum entsprechenden 4-$R_4$-5-$R_3$-4,5-dihydro-[1,2,4]-triazolo[1,5-a]chinoxalin-2-methanol der allgemeinen Formel II reduziert werden. Letzteres kann in üblicher Weise in den zur Umsetzung gemäss a) gewünschten reaktionsfähigen Ester übergeführt werden, z.B. durch Umsetzung mit Methansulfonylchlorid in Gegenwart einer geeigneten Base, z.B. Triäthylamin, in einem inerten organischen Lösungsmittel in den entsprechenden Methansulfonsäureester, oder durch Umsetzung mit Phosphortribromid in einem inerten organischen Lösungsmittel in das entsprechende Bromid, d.h. die entsprechende 2-(Brommethyl)-verbindung.

Verbindungen der allgemeinen Formel II, in denen Ar einen Pyridorest bedeutet, können analog hergestellt werden, doch gelangt man zu solchen Verbindungen, in denen Ar ein Pyrido[2,3-e]-rest ist und die weder in 7- noch in 9-Stellung durch Chlor oder Brom substituiert sind, meist vorteilhafter durch nachstehende Reaktionsfolge ausgehend von gegebenenfalls, jedoch nicht durch Chlor oder vor allem Brom in 4- oder 6-Stellung substituiertem 3-Amino-2-chlorpyridin. Die genannte Aminoverbindung wird in üblicher Weise in das entsprechende Diazoniumchlorid übergeführt und letzteres mit einem (2-Chlorniederalkanamido)-malonsäurediniederalkylester, wie dem Diäthylester oder Dimethylester zum entsprechenden (2-Chlorniederalkanamido)-(2-chlor-3-pydridylazo)-malonsäurediniederalkylester gekuppelt. Dieser wird durch aufeinanderfolgende Behandlung mit wässrig-alkanolischer Natriumhydroxidlösung bei Raumtemperatur und mit Salzsäure in der Kälte in die entsprechende 1-(2-Chlor-3-pyridyl)-5-(1-chlorniederalkyl)-1H-1,2,4-triazol-3-carbonsäure übergeführt. Letztere wird durch Umsetzung mit Ammoniak oder einem primären Amin der Formel $R_3$-$NH_2$, gegebenenfalls in Gegenwart einer katalytischen bis doppeltmolaren Menge eines Alkalimetalljodids in die entsprechende 4-$R_4$-5-$R_3$-4,5-dihydro-pyrido[2,3-b]-[1,2,4]triazolo[1,5-d]pyrazin-2-carbonsäure übergeführt und letztere analog zur vorgenannten Reaktionsfolge, vorzugsweise nach Veresterung, zur entsprechenden Verbindung der allgemeinen Formel II reduziert und diese in einen geeigneten reaktionsfähigen Ester übergeführt.

Verbindungen der allgemeinen Formel II, in denen sowohl $R_4$ als auch $R_5$ Niederalkyl, insbesondere Methyl, bedeuten, erhält man beispielsweise dadurch, dass man einen in den obengenannten Reaktionsfolgen als unmittelbare Vorstufe zur Hydroxyverbindung der allgemeinen Formel II erhaltenen Ester, z.B. Methylester, in welchem $R_4$ Niederalkyl, insbesondere Methyl ist, nicht sogleich reduziert, sondern zuvor, z.B. mittels Lithium-diisopropylamid, in die entsprechende Alkalimetallverbindung überführt und letztere mit einem Niederalkylhalogenid, wie Methyljodid, umsetzt, und erst anschliessend die Estergruppe zur Hydroxymethylgruppe reduziert.

Die Ausgangsstoffe der allgemeinen Formel IV, in denen $X_1$ Sauerstoff und n Null ist, für die Reduktion gemäss b) sind ebenfalls neu. Sie können aus den entsprechenden, vorstehend genannten Carbonsäuren oder deren funktionellen Derivaten, z.B. den ebenfalls bereits genannten Niederalkylestern oder z.B. auch den entsprechenden Säurechloriden, durch Umsetzung mit Aminen der allgemeinen Formel III in an sich bekannter Weise hergestellt werden. Solche Ausgangsstoffe der allgemeinen Formel IV, in denen $R_4$ und $R_5$ je Niederalkyl, insbesondere Methyl bedeuten, stellt man vorzugsweise aus entsprechenden Verbindungen mit Niederalkyl als $R_4$ und Wasserstoff als $R_5$ durch Einführung von Niederalkyl $R_5$ analog zur vorstehend angegebenen Einführung von Niederalkyl $R_5$ in die den Amiden der allgemeinen Formel IV entsprechenden Ester her. Verbindungen der allgemeinen Formel IV, in denen X zwei Wasserstoffatome und n 1 bedeutet, erhält man aus Verbindungen der allgemeinen Formel I, in denen $R_1$ und gegebenenfalls auch $R_2$ Wasserstoff bedeutet, durch Einführung eines Niederalkanoyl-, Hydroxyniederalkanoyl- oder Niederalkoxycarbonylrestes, z.B. durch Umsetzung mit einem Niederalkylester der entsprechenden Niederalkansäure oder Hydroxyniederalkansäure,oder mit einem Niederalkanoylchlorid oder Chlorameisensäureniederalkylester.

Die neuen Verbindungen können, je nach der Wahl der Ausgangsstoffe und Arbeitsweisen, in Form eines der möglichen Stereoisomeren oder als Gemisch derselben, z.B. je nach der Anzahl der Asymmetriezentren als reine optische Isomere, d.h. optische Antipoden oder als Racemate, und im Falle von Diastereoisomerie auch als Racematgemische, vorliegen.

Erhaltene Racematgemische können auf Grund der physikalisch-chemischen Unterschiede der Bestandteile in bekannter Weise in die reinen Racemate aufgetrennt werden, beispielsweise durch Chromatographie und/oder fraktionierte Kristallisation.

Erhaltene Racemate lassen sich nach bekannten Methoden in die optischen Antipoden zerlegen, beispielsweise durch Umkristallisation aus einem optisch aktiven Lösungsmittel, mit Hilfe von Mikroorganismen, oder durch Umsetzen eines Endstoffes mit einer mit der racemischen Base Salze bildenden optisch aktiven Säure und Trennung der auf diese Weise erhaltenen Salze, z.B. auf Grund ihrer verschiedenen Löslichkeiten, in die Diastereomeren, aus denen die Antipoden durch Einwirkung geeigneter Mittel freigesetzt werden können. Vorteilhaft isoliert man den wirksameren der beiden Antipoden.

Erhaltene freie Verbindungen der Formel I können in an sich bekannter Weise in Säureadditionssalze übergeführt werden, u.a. durch Behandeln mit der entsprechenden Säure, üblicherweise in Gegenwart eines Lösungs- oder Verdünnungsmittels.

Erhaltene Säureadditionssalze können in an sich bekannter Weise in die freien Verbindungen umgewandelt werden, z.B. durch Behandeln mit einer Base, wie einem Alkalimetallhydroxid oder einem basischen Ionenaustauscher.

Die Verbindungen einschliesslich ihrer Salze können auch in Form ihrer Hydrate erhalten werden oder das zur Kristallisation verwendete Lösungsmittel einschliessen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder als Ausgangsstoff anstelle eines Racemats einen optischen Antipoden und/oder einen Ausgangsstoff in Form eines Salzes verwendet oder insbesondere unter den Reaktionsbedingungen bildet.
Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu ihrer Herstellung bilden ebenfalls einen Gegenstand der vorliegenden Erfindung.

Die erfindungsgemässen pharmazeutischen Präparate enthalten z.B. von etwa 10% bis etwa 95%, vorzugsweise von etwa 20% bis etwa 90% an mindestens einem erfindungsgemässen Wirkstoff zusammen mit mindestens einem pharmazeutischen Träger- oder Hilfsstoff. Erfindungsgemässe pharmazeutische Präparate sind z.B. solche in Dosiseinheitsformen, wie Dragées, Tabletten, Kapseln oder Suppositorien, ferner Ampullen, Pro Dosiseinheit enthalten solche Präparate z.B. 10 bis 500 mg, und vorzugsweise 25-250 mg Wirkstoff.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt.

So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet. Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, z.B. von Mais-, Weizen-, Reis- oder Kartoffelstärke, Gelatine, Tragacanth, Methylcellulose und/oder Polyvinylpyrrolidon, und/oder, wenn erwünscht, Sprengmittel, wie die obengenannten Stärken ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den

Wirkstoff in Form eines Granulates, z.B. im Gemisch mit Füllstoffen, wie Lactose, Bindemitteln, wie Stärken und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässerige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls auch Stabilisatoren enthalten.

Die Erfindung betrifft ebenfalls die Verwendung der Verbindungen der allgemeinen Formel I und ihrer pharmazeutisch annehmbaren Säureadditionssalze als Arzneimittel, insbesondere als Antidepressiva oder Anxiolytica, vorzugsweise in der Form von pharmazeutischen Präparaten. Die Dosierung hängt von der Warmblüter-Spezies, dem Alter und dem individuellen Zustand des zu behandelnden Warmblüters sowie von der Applikationsweise ab. Die pro Tag verabreichten Dosen liegen zwischen etwa 1 und etwa 100 mg/kg und vorzugsweise, z.B. für Warmblüter von ca. 70 kg Körpergewicht, zwischen etwa 3 und etwa 50 mg/kg.

Die nachfolgenden Beispiele illustrieren die oben beschriebene Erfindung; sie sollen diese jedoch in ihrem Umfang in keiner Weise einschränken. Temperaturen werden in Celsiusgraden angegeben.

Beispiel 1: Zu der Suspension von 5,00 g (0,018 Mol) 5-Phenyl-4,5-dihydro-[1,2,4]-triazolo[1,5-a]chinoxalin-2-methanol in 180 ml abs. Methylenchlorid und 3,64 g (0,036 Mol) Triäthylamin werden unter Stickstoff bei -10° 3,10 g (0,027 Mol) Methansulfonsäurechlorid innerhalb 5 Minuten zugetropft. Nach einstündigem Rühren bei 0° gibt man nochmals 0,70 g Triäthylamin und 0,60 g Methansulfochlorid zu, rührt noch weitere 30 Minuten bei 0° und dampft die klare Reaktionslösung im Vakuum bei 35° vollständig ein, wobei der Methansulfonsäureester des 5-Phenyl-4,5-dihydro-[1,2,4]-triazolo[1,5-a]chinoxalin-2-methanols als farbloser kristalliner Rückstand (enthält Triäthylamin-hydrochlorid) erhalten wird. Der so erhaltene Methansulfonsäureester wird ohne weitere Reinigung sofort weiter umgesetzt, indem man ein eiskaltes Gemisch von 15,0 ml (0,11 Mol) 33%-iger äthanolischer Dimethylaminlösung und 15 ml Aethanol zugibt und das Reaktionsgemisch verschlossen während einer Stunde rührt. Hierauf dampft man es im Vakuum ein und löst den Rückstand in Aethylacetat und 1-n. Kaliumbicarbonatlösung. Die wässrige Phase wird abgetrennt und noch zweimal mit Aethylacetat extrahiert. Die vereinigten organischen Extrakte werden dreimal mit Wasser und einmal mit gesättigter Natriumchloridlösung gewaschen und über Natriumsulfat getrocknet. Nach Abfiltrieren des Trocknungsmittels versetzt man das Filtrat unter gutem Rühren tropfenweise mit ätherischer Chlorwasserstofflösung, bis der pH-Wert 4 erreicht. Nach 3-stündigem Rühren im Eisbad wird das gebildete rohe Hydrochlorid abfiltriert und mit Aethylacetat gewaschen. Hierauf wird es in 70 ml Methylenchlorid und 100 ml Isopropanol gelöst. Nach dem Abdampfen des Methylenchlorids im Vakuum bei 40° kristallisiert das gewünschte Hydrochlorid wieder aus. Man lässt es etwa 15 Stunden im Eisschrank stehen, filtriert die Kristalle ab und wäscht sie mit Isopropanol. Nach dem Trocknen im Hochvakuum bei 120° schmilzt das erhaltene 2-[(Dimethylamino)-methyl]-5-phenyl-4,5-dihydro-[1,2,4]-triazolo[1,5-a]-chinoxalin-hydrochlorid der Formel

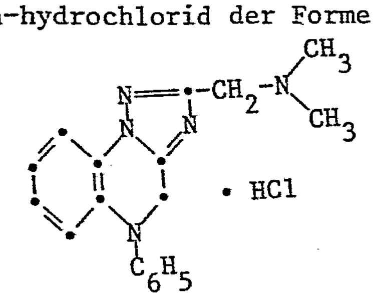

bei 232-235° unter Zersetzung.

Das als Ausgangsstoff benötigte 5-Phenyl-4,5-dihydro-[1,2,4]-triazolo [1,5-a]chinoxalin-2-methanol wird wie folgt hergestellt:

a) Zu einer Suspension von 19,60 g (ca. 0,41 Mol) Natriumhydrid/Mineralöl in 100 ml Dimethylformamid wird bei 10 -15° unter Stickstoff eine Lösung von 79,40 g (0,371 Mol) o-Nitrodiphenylamin [vgl. F. Kehrmann und E. Havas, Ber. 46, 341 (1913)] in 500 ml Dimethylformamid innerhalb einer Stunde unter gutem Rühren zugetropft. Man rührt das Reaktionsgemisch noch drei Stunden bei Raumtemperatur und tropft dann 45,0 ml (0,472 Mol) Chlorameisensäureäthylester bei 20° zu. Nach einstündigem Rühren bei Raumtemperatur kühlt man das Reaktionsgemisch auf 0° ab und tropft langsam 15 ml Eisessig zu, wobei der pH-Wert am Schluss 5 erreicht. Hierauf giesst man das Reaktionsgemisch auf Eis-Wasser-Gemisch und extrahiert dreimal mit Aethylacetat. Die Extrakte werden dreimal mit Wasser und zweimal mit gesättigter Natriumchloridlösung gewaschen, über Natriumsulfat getrocknet und im Vakuum zur Trockne eingedampft. Der Rückstand wird mit 200 ml Hexan und 100 ml Wasser während drei Stunden verrührt. Das auskristallisierte Rohprodukt wird abfiltriert und gut mit Wasser und Hexan gewaschen. Nach Umkristallisieren aus Isopropanol erhält man den (2-Nitrophenyl)-phenylcarbaminsäure-äthylester vom Smp. 53-56°.

b) Eine Lösung von 35,15 g (0,123 Mol) (2-Nitrophenyl)-phenylcarbaminsäure-äthylester in 350 ml Aethanol wird unter Zusatz von 8,0 g Raney-Nickel bei Normaldruck während 25 Stunden bei 15-20° hydriert. Nach Abfiltrieren des Katalysators wird das Filtrat im Vakuum bei maximal 40° eingedampft und der Tückstand aus Methylenchlorid/Hexan umkristallisiert. Nach dem Trocknen im Vakuum schmilzt der erhaltene N-(2-Aminophenyl)--phenylcarbaminsäure-äthylester bei 87-90°.

c) Eine Lösung von 34,00 g (0,133 Mol) (2-Aminophenyl)-phenylcarbaminsäure-äthylester in 319 ml Eisessig und 80 ml konz. Salzsäure

wird bei 0-5° mit 27 ml (0,133 Mol) einer 5-molaren Natriumnitritlösung diazotiert. Die erhaltene Diazoniumsalzlösung versetzt man mit 80 g Eis und tropfenweise rasch mit einer Lösung von 33,40 g (0,133 Mol) (2-Chloracetamido)-malonsäure-diäthylester [vgl. Ajay Kumar Bose, J. Indian Chem. Soc. 31, 108 - 110 (1954)] in 330 ml Aceton. Anschliessend tropft man bei 0 - 5° in Laufe von 30 Minuten 610 ml einer gesättigten Kaliumcarbonatlösung zu, wobei der pH-Wert des Reaktionsgemisches am Schluss 6 erreicht. Nach Zugabe von 50 ml Aether rührt man 90 Minuten im Eisbad und filtriert dann das bereits jetzt auskristallisierte Reaktionsprodukt. Die Kristalle werden gut mit Wasser und wenig Aether gewaschen. Nach dem Trocknen über Calciumchlorid im Exsikkator schmilzt der erhaltene [2-(N-Aethoxy-carbonyl-phenylamino)-phenylazo]-(2-chloracetamido)-malonsäure-diäthylester bei 102-104°. Zur Gewinnung von weiterem Reaktionsprodukt werden die vereinigten Filtrate mit 500 ml Aethylacetat extrahiert. Die abgetrennte organische Phase wird abgetrennt, viermal mit Wasser und einmal mit gesättigter Natriumchloridlösung gewaschen, über Natriumsulfat getrocknet und im Vakuum eingedampft. Den Rückstand löst man in 50 ml Aether und lässt zur Kristallisation zwei Tage bei Raumtemperatur verschlossen stehen. Hierauf werden die entstandenen Kristalle abfiltriert und mit wenig Aether und viel Hexan gewaschen. Nach dem Trocknen erhält man eine die direkt gewonnene übertreffende Menge des gleichen Produktes vom Schmelzpunkt 102-104°.

d) Zu 848 ml (0,848 Mol) einer auf 0° vorgekühlten 1-n. Natriumhydroxidlösung gibt man unter gutem Rühren innerhalb 5 Minuten eine Lösung von 146,6 g (0,283 Mol) der nach c) hergestellten Azoverbindung in 1466 ml Methanol. Man rührt 90 Minuten bei Raumtemperatur aus und tropft anschliessend 450 ml 2-n. Salzsäure unter Kühlung mit Eis-Wasser-Gemisch bis zur kongosauren Reaktion zu. Die freie Carbonsäure kristallisiert nach Animpfen und Reiben mit dem Glasstab aus. Der entstehende Kristallbrei wird 3 Stunden bei 0° gerührt, dann abgesaugt, mit Wasser bis pH 5 gewaschen und über Calciumchlorid im

Vakuum bei 80° getrocknet. Die erhaltene 1-[2-(N-Aethoxycarbonyl-phenylamino)-phenyl]-5-(chlormethyl)-1H-1,2,4,-triazol-3-carbonsäure sintert bei 158° und schmilzt unter Zersetzung bei 167°-170°.

e) Eine Suspension von 53,80 g (0,134 Mol) der nach d) hergestellten Carbonsäure in 280 ml 48%-iger Bromwasserstoffsäure wird während 22 Stunden bei einer Badtemperatur von 105° unter Stickstoff gerührt, wobei zunächst eine klare Lösung entsteht und nach zwei Stunden allmählich eine kristalline Fällung erscheint. Anschliessend kühlt man das Reaktionsgemisch auf Raumtemperatur ab und fügt unter Rühren 300 ml Wasser zu. Nach 3-stündigem Kühlen auf 0° wird die Carbonsäure abgesaugt, mit total 800 ml Wasser bis pH 5 gewaschen und im Vakuum zunächst über Calciumchlorid und dann über Phosporpentoxid getrocknet. Die erhaltene 5-Phenyl-4,5-dihydro-[1,2,4]-triazolo[1,5-a]chinoxalin-2-carbonsäure schmilzt bei 205-207° unter Zersetzung.

f) Eine Suspension von 74,90 g (0,256 Mol) der nach e) hergestellten Carbonsäure in 1500 ml Methanol und 300 ml 6-n. methanolischer Salzsäurelösung wird unter Stickstoff 17 Stunden bei Siedetemperatur unter Rückfluss gerührt, wobei nach einer Stunde eine klare Lösung vorliegt. Anschliessend rührt man das Reaktionsgemisch 4 Stunden bei 0°, saugt die gebildeten Kristalle ab und wäscht sie mit total 250 ml Methanol. Nach dem Trocknen über Calciumchlorid und Kaliumhydroxid im Vakuum sintert der erhaltene 5-Phenyl-4,5-dihydro-[1,2,4]-triazolo-[1,5-a]chinoxalin-2-carbonsäure-methylester bei 140° und schmilzt bei 150-154°.

g) Eine Suspension von 87,0 g (0,285 Mol) des nach f) hergestellten Esters in 870 ml Methanol und 870 ml Tetrahydrofuran wird unter Rühren und Stickstoff bei Raumtemperatur innerhalb 15 Minuten mit total 30,0 g (0,794 Mol) Natriumborhydrid, verteilt auf drei Portionen, versetzt. Unter starkem Schäumen steigt die Temperatur des

Reaktionsgemisches dabei auf 30° an und wird durch Kühlung mit kaltem Wasser dort gehalten. Man rührt 2 Stunden bei Raumtemperatur udn nach Zusatz von 1750 ml Eis-Wasser-Gemisch noch 3 Stunden bei 0°. Der ausgefallene Alkohol wird abgesaugt und mit Wasser bis pH 6 gewaschen. Nach dem Trocknen über Calciumchlorid im Vakuum schmilzt das erhaltene 5-Phenyl-4,5-dihydro-[1,2,4]-triazolo[1,5-a]chinoxalin-2-methanol bei 180 - 185°.

Beispiel 2: Den aus 8,70 g (0,0313 Mol) 5-Phenyl-4,5-dihydro-[1,2,4]-triazolo[1,5-a]chinoxalin-2-methanol und 5,92 g (0,051 Mol) Methansulfonsäurechlorid gemäss Beispiel 1 bereiteten rohen Methansulfonsäureester rührt man nach Zusatz von 24 ml (0,188 Mol) 33 %-iger äthanolischer Methylaminlösung und 24 ml Aethanol eine Stunde bei Raumtemperatur. Hierauf dampft man das Reaktionsgemisch im Vakuum ein und löst den Rückstand in Aethylacetat und 1-n. Kaliumbicarbonatlösung. Die wässrige Phase wird abgetrennt und noch zweimal mit Aethylacetat extrahiert. Die vereinigten organischen Extrakte werden 3-mal mit Wasser und einmal mit gesättigter Natriumchloridlösung gewaschen, über Natriumsulfat getrocknet und im Vakuum zur Trockne eingedampft.

Den Rückstand von 9,20 g löst man in Methylenchlorid-Methanol (9:1) und chromatographiert die Lösung an einer Säule von 900 g Kieselgel. Als Elutionsmittel verwendet man ebenfalls Methylenchlorid-Methanol (9:1). Die Fraktionen, welche das gewünschte Rohprodukt vom Rf-Wert 0,4 [System: Methylenchlorid-Methanol (9:1)] enthalten, werden zur Trockne eingedampft, in 30 ml Aceton gelöst und mit einer Lösung von Oxalsäure in Aceton versetzt, bis der pH-Wert 6 erreicht. Man lässt die Lösung etwa 15 Stunden bei Raumtemperatur stehen, filtriert die entstandenen Kristalle ab und wäscht sie mit Aceton. Nach dem Trocknen im Hochvakuum bei 90° sintert das erhaltene 2-[(Methylamino)-methyl]-5-phenyl-4,5-dihydro-[1,2,4]-triazolo[1,5-a]chinoxalin-oxalat bei 190° und schmilzt bei 210 - 213°.

Beispiel 3: Eine Suspension von 22,50 g (0,066 Mol) 2-(Brommethyl)-5-phenyl-4,5-dihydro-[1,2,4]-triazolo[1,5-a]chinoxalin in 225 ml Isopropanol und 45,30 ml (0,33 Mol) 33 %-ige äthanolischer Dimethylaminlösung wird unter Stickstoff bei Raumtemperatur zwei Stunden gerührt. Hierauf dampft man im Vakuum ein und löst den Rückstand in Aethylacetat und 1-n. Kaliumbicarbonatlösung. Die wässrige Phase wird abgetrennt und noch zweimal mit Aethylacetat extrahiert. Die vereinigten organischen Extrakte werden dreimal mit Wasser und einmal mit gesättigter Natriumchloridlösung gewaschen und über Natriumsulfat getrocknet. Das Trocknungsmittel wird abfiltriert und das Filtrat unter gutem Rühren tropfenweise mit ätherischer Chlorwasserstofflösung versetzt, bis der pH-Wert 4 erreicht. Nach 3-stündigem Rühren im Eisbad wird das gebildete rohe Hydrochlorid abfiltriert und mit Aethylacetat gewaschen. Die Umkristallisation wie im Beispiel 1 beschrieben ergibt das 2-[(Dimethylamino)-methyl]-5-phenyl-4,5-dihydro-[1,2,4]-triazolo[1,5-a]chinoxalin-hydrochlorid vom Smp. 232-235 unter Zersetzung.

Analog erhält man aus 10,0 g (0,029 Mol) derselben Brommethylverbindung mit 10,70 g (0,146 Mol) Diäthylamin das 2-[(Diäthylamino)-methyl]-5-phenyl-4,5-dihydro-[1,2,4]-triazolo[1,5-a]chinoxalin-hydrochlorid vom Smp. 192-195° (unter Zersetzung) nach Kristallisation aus Methylenchlorid/Aethylacetat und mit 12,40 g (0,146 Mol) Piperidin das 2-[(Piperidino)-methyl]-5-phenyl-4,5-dihydro-[1,2,4]-triazolo-[1,5-a]chinoxalin-hydrochlorid vom Smp. 240-250° (unter Zersetzung) nach Kristallisation aus Methylenchlorid/Aethylacetat.

Das als Ausgangsstoff benötigte 2-(Brommethyl)-5-phenyl-4,5-dihydro-[1,2,4]-triazolo[1,5-a]chinoxalin wird wie folgt hergestellt:

a) In die Suspension von 44,30 g (0,159 Mol) des gemäss Beispiel 1 g) bereiteten 5-Phenyl-4,5-dihydro-[1,2,4]-triazolo[1,5-a]chinoxalin-2-methanols in 660 ml abs. Methylenchlorid tropft man bei 20° unter

Stickstoff 43,00 g (0,159 Mol) Phosphortribromid innerhalb 30 Minuten zu und rührt noch 4 Stunden bei Raumtemperatur aus. Hierauf dampft man im Vakuum bei 40° zur Trockne ein und löst den Rückstand in 1200 ml Aethylacetat und 200 ml Eis-Wasser-Gemisch. Die wässrige Phase wird abgetrennt und noch einmal mit Aethylacetat extrahiert. Die vereinigten organischen Extrakte werden 4-mal mit Wasser und einmal mit gesättigter Natriumchloridlösung gewaschen, über Natriumsulfat getrocknet und im Vakuum zur Trockne eingedampft. Der kristalline Rückstand wird in 200 ml Methylenchlorid und 500 ml Isopropanol gelöst. Nach dem Abdampfen des Methylenchlorids im Vakuum bei 40° kristallisiert die gewünschte Verbindung aus. Man lässt den Kristallbrei etwa 15 Stunden im Eisschrank stehen, filtriert die Kristalle ab und wäscht sie mit Isopropanol. Nach dem Trocknen über Calciumchlorid im Vakuumexsikkator sintert das erhaltene 2-(Brommethyl)-5-phenyl-4,5-dihydro-[1,2,4]-triazolo[1,5-a]chinoxalin ab 115° und schmilzt bei 122-130°.

<u>Beispiel 4:</u> In die Suspension von 1,20 g (0,0037 Mol) N,N-Dimethyl-5-phenyl-4,5-dihydro-[1,2,4]-triazolo[1,5-a]chinoxalin-2-carboxamid in 180 ml abs. Toluol tropft man bei 2° unter Stickstoff 9,40 ml (0,011 Mol) einer 1,2-molaren Lösung von Diisobutylaluminiumhydrid in Toluol innterhalb 15 Minuten zu. Die entstandene klare gelbe Lösung rührt man noch eine Stunde bei 5°, tropft dann 5 ml Isopropanol in das Reaktionsgemisch und giesst es auf Eis-Wasser-Gemisch. Die organische Phase wird abgetrennt, zweimal mit Eiswasser und einmal mit gesättigter Natriumchloridlösung gewaschen,über Natriumsulfat getrocknet und im Vakuum eingedampft. Den honigartigen Rückstand löst man in 20 ml Aethylacetat und tropft unter gutem Rühren langsam ätherische Chlorwasserstofflösung zu, bis der pH-Wert 4 erreicht. Nach etwa 15 Stunden Stehenlassen im Eisschrank wird das rohe Hydrochlorid abfiltriert und mit Aethylacetat gewaschen. Umkristallisation wie im Beispiel 1 beschrieben ergibt das 2-[(Dimethylamino)-methyl]-5-phenyl-4,5-dihydro-[1,2,4]-triazolo[1,5-a]-chinoxalin-hydrochlorid vom Smp. 232-235° unter Zersetzung.

Das als Ausgangsstoff benötigte Carboxamid wird wie folgt hergestellt:

a) Das Gemisch von 7,00 g (0,023 Mol) des gemäss Beispiel 1f) hergestelltem 5-Phenyl-4,5-dihydro-[1,2,4]-triazolo[1,5-a]chinoxalin-2-carbonsäure-methylesters in 300 ml Methanol und 62,6 ml (0,457 Mol) 33 %-iger äthanolischer Dimethylaminlösung wird in einem Autoklaven 16 Stunden auf 100° erhitzt. Hierauf dampft man das Reaktionsgemisch im Vakuum ein und löst den Rückstand in Methylenchlorid und Eis-Wasser-Gemisch. Die organische Phase wird zweimal mit kalter 5%-iger Kaliumbicarbonatlösung und einmal mit gesättigter Natriumchloridlösung gewaschen, über Natriumsulfat getrocknet und im Vakuum eingedampft. Der amorphe Rückstand kristallisiert beim Anreiben mit 300 ml Aether durch. Nach Filtration und Trocknen im Hochvakuum erhält man das N,N-Dimethyl-5-phenyl-4,5-dihydro-[1,2,4]-triazolo[1,5-a]chinoxalin-2-carboxamid, das ab 125° sintert und bei 150 - 165° allmählich schmilzt.

Beispiel 5: Analog Beispiel 1 erhält man ausgehend von 9,30 g (0,0297 Mol) 5-(4-Chlorphenyl)-4,5-dihydro-[1,2,4]-triazolo[1,5-a]-chinoxalin-2-methanol über dessen rohen Methansulfonsäureester unter Verwendung von 40,7 ml (0,297 Mol) 33 %-iger äthanolischer Dimethylaminlösung das 2-[(Dimethylamino)-methyl]-5-(4-chlorphenyl)-4,5-dihydro-[1,2,4]-triazolo[1,5-a]chinoxalin-hydrochlorid, das nach Umkristallisation aus Methylenchlorid/Isopropanol bei 230° sintert und bei 248-251° unter Zersetzung schmilzt.

Analog Beispiel 1 erhält man ausgehend von 20,30 g (0,059 Mol) 5-[3-(Trifluormethyl)-phenyl]-4,5-dihydro-[1,2,4]-triazolo[1,5-a]-chinoxalin-2-methanol über dessen rohen Methansulfonsäureester unter Verwendung von 48,2 ml (0,35 Mol) 33%-iger äthanolischer Dimethylaminlösung das 2-[(Dimethylamino)-methyl]-5-[3-(trifluormethyl)-phenyl]-4,5-dihydro-[1,2,4]-triazolo[1,5-a]chinoxalin, das zur Reinigung an basischem Aluminiumoxid der Aktivitätsstufe I mit dem Elutionsmittel Methylenchlorid-Methanol (99:1) chromatographiert und anschliessend aus Hexan umkristallisiert wird. Das Produkt (freie Base) schmilzt dann bei 115-118°.

Analog Beispiel 1 erhält man ausgehend von 35,05 g (0,144 Mol) 5-(2,3-Dimethylphenyl)-4,5-dihydro-[1,2,4]-triazolo[1,5-a]chinoxalin-2-methanol über dessen rohen Methansulfonsäureester unter Verwendung von 198 ml (1,44 Mol) 33%-iger äthanolischer Dimethylaminlösung das 2-[(Dimethylamino)-methyl]-5-(2,3-dimethylphenyl)-4,5-dihydro-[1,2,4]-triazolo[1,5-a]chinoxalin-hydrochlorid, das nach Umkristallisation aus Methylenchlorid/Isopropanol bei 250° sintert und bei 257-259° unter Zersetzung schmilzt.

Analog Beispiel 1 erhält man ausgehend von 8,0 g (0,026 Mol) 7-Chlor-5-phenyl-4,5-dihydro- [1,2,4]-triazolo[1,5-a]chinoxalin-2-methanol über dessen rohen Methansulfonsäureester unter Verwendung von 25,6 ml (0,218 Mol) 33 %-iger äthanolischer Dimethylaminlösung das 7-Chlor-2-[(dimethylamino)-methyl]-5-phenyl-4,5-dihydro- [1,2,4]-triazolo[1,5-a]-chinoxalin-hydrochlorid, das nach Umkristallisation aus Methylenchlorid/Aethylacetat ab 220° sintert und bei 228 - 232° unter Zersetzung schmilzt.

Analog Beispiel 1 erhält man ausgehend von 16,50 g (0,0432 Mol) 8-Chlor-5-(2,4-dichlorphenyl)-4,5-dihydro- [1,2,4]-triazolo[1,5-a]-chinoxalin-2-methanol über dessen rohen Methansulfonsäureester unter Verwendung von 59,0 ml (0,432 Mol) 33%-iger äthanolischer Dimethylaminlösung das 8-Chlor-2-[(dimethylamino)-methyl]-5-(2,4-dichlorphenyl)-4,5-dihydro- [1,2,4]-triazolo[1,5-a]chinoxalin-hydrochlorid, das nach Umkristallisation aus Methylenchlorid/Isopropanol ab 282°

unter Zersetzung schmilzt.

Die als Ausgangstoffe benötigten Methanol-Derivate werden wie folgt hergestellt:

a) Analog Beispiel 1a) erhält man unter Verwendung von 26,2 g (0,105 Mol) 4-Chlor-N-(2-nitrophenyl)-anilin [vgl. V.C. Barry et al., Proc. Roy. Irish Acad., 55 B, 160 (1953)] den (4-Chlorphenyl)-(2-nitrophenyl)-carbaminsäure-äthylester vom Smp. 90-93° nach Umkristallisation aus Methylenchlorid/Hexan;

unter Verwendung von 95,08 g (0,337 Mol) 2-Nitro-N-[3-(trifluormethyl)-phenyl]-anilin [vgl. nachstehenden Abschnitt h)] den (2-Nitrophenyl)-[3-(trifluormethyl)-phenyl]-carbaminsäure-äthylester vom Smp. 45-48° nach Umkristallisation aus Hexan;

unter Verwendung von 65,0 g (0,268 Mol) 2,3-Dimethyl-N-(2-nitrophenyl)-anilin [vgl. nachstehenden Abschnitt h)] den (2,3-Dimethylphenyl)-(2-nitrophenyl)-carbaminsäure-äthylester vom Smp. 105 - 106° nach Umkristallisation aus Isopropanol;

unter Verwendung von 43,00 g (0,173 Mol) 5-Chlor-2-nitro-N-phenyl-anilin [vgl. A.P.Kottenhahn et al., J.Org. Chem. 28, 3117 (1963)] nach Chromatographie des Rohproduktes an Kieselgel mit dem Elutionsmittel Toluol den (5-Chlor-2-nitro-phenyl)-phenylcarbaminsäure-äthylester als braunes Oel, das sich nach einiger Zeit verfestigt und dann bei 71 - 73° schmilzt;

unter Verwendung von 56,55 g (0,178 Mol) 2,4-Dichlor-N-(2-nitrophenyl)-anilin [vgl. V.C. Barry und J.G. Belton, Proc. Roy. Irish Acad., 57B, 144 (1955)] nach Chromatographie des Rohproduktes an Kieselgel mit dem Elutionsmittel Toluol den (4-Chlor-2-nitrophenyl)-(2,4-dichlorphenyl)-carbaminsäure-äthylester vom Smp. 93°-96° nach

Umkristallisation aus Isopropanol.

b) Analog Beispiel 1b) erhält man unter Verwendung von 41,20 g (0.128 Mol) (4-Chlorphenyl)-2-nitrophenyl)-carbaminsäure-äthylester den (2-Aminophenyl)-(4-chlorphenyl)-carbaminsäure-äthylester vom Smp. 112-115° nach Umkristallisation aus Methylenchlorid/Hexan;

unter Verwendung von 81,50 g (0,230 Mol) 2-Nitrophenyl-[3-(trifluormethyl)-phenyl]-carbaminsäure-äthylester den (2-Aminophenyl)-[3-(trifluormethyl)-phenyl]-carbaminsäure-äthylester vom Smp. 105-108° nach Umkristallisation aus Methylenchlorid/Hexan;

unter Verwendung von 71,90 g (0,229 Mol) (2,3-Dimethylphenyl)-(2-nitrophenyl)-carbaminsäureäthylester den (o-Aminophenyl)-(2,3-dimethylphenyl)-carbaminsäure-äthylester vom Smp. 95-97° nach Umkristallisation aus Methylenchlorid/Hexan;

unter Verwendung von 43,20 g (0,135 Mol) 5-Chlor-2-nitrophenyl-phenylcarbaminsäure-äthylester den (2-Amino-5-chlor-phenyl)-phenylcarbaminsäure-äthylester vom Smp. 98-101° nach Umkristallisation aus Methylenchlorid/Hexan;

unter Verwendung von 39,85 g (0,102 Mol) (2,4-Dichlorphenyl)-(4-chlor-2-nitrophenyl)-carbaminsäure-äthylester den (2-Amino-4-chlor-phenyl)-(2,4-dichlorphenyl)-carbaminsäure-äthylester vom Smp. 137-139° nach Umkristallisation aus Methylenchlorid/Hexan.

c) Analog Beispiel 1c) erhält man unter Verwendung von 38,60 g (0,133 Mol) (2-Amino-phenyl)-(4-chlorphenyl)-carbaminsäure-äthylester den [2-(N-Aethoxycarbonyl-4-chlorphenylamino)-phenylazo]-(2-chloracetamido)-malonsäure-diäthylester vom Smp. 80 - 85° nach Umkristallisation aus Aether /Hexan;

unter Verwendung von 61,20 g (0,189 Mol) (2-Aminophenyl)-[3-(trifluormethyl)-phenyl]-carbaminsäure-äthylester den [2-[N-Aethoxycarbonyl-3-(trifluormethyl)-phenylamino]-phenylazo]-(2-chloracetamido)-malonsäure-diäthylester vom Smp. 76 - 79° nach Umkristallisation aus Aether/Hexan;

unter Verwendung von 56,60 g (1,199 Mol) (2-Amino-phenyl)-(2,3-Dimethylphenyl)-carbaminsäure-äthylester den [2-(N-Aethoxycarbonyl-2,3-dimethylphenylamino)-phenylazo]-(2-chloracetamido)-malonsäure-diäthylester vom Smp. 120-122° nach Umkristallisation aus Aether/Hexan.

unter Verwendung von 31,60 g (0,109 Mol) (2-Amino-5-chlor-phenyl)-phenylcarbaminsäure-äthylester den [2-(N-Aethoxycarbonyl-phenyl-amino)-4-chlor-phenylazo]-(2-chloracetamido)-malonsäure-diäthylester, der nach Kristallisation aus Aether ab 90° sintert und bei 105-110° schmilzt;

unter Verwendung von 33,30 g (0,093 Mol) (2-Amino-4-chlorphenyl)-(2,4-dichlorphenyl)-carbaminsäure-äthylester den amorphen [2-(N-Aethoxycarbonyl-2,4-dichlor-phenylamino)-5-chlor-phenylazo]-(2-chloracetamido)-malonsäure-diäthylester. Dieser rohe Malonester kann direkt zur Cyclisierung analog Beispiel 1d) eingesetzt werden.

d) Analog Beispiel 1d) erhält man unter Verwendung von 36,90 g (0,067 Mol) [2-(N-Aethoxycarbonyl-4-chlor-phenylamino)-phenylazo]-(2-chloracetamido)-malonsäure-diäthylester die rohe 1-[2-(N-Aethoxycarbonyl-4-chlor-phenylamino)-phenyl]-5-(chlormethyl)-1H-1,2,4-triazol-3-carbonsäure, die bei 185° sintert und bei 190-192° unter Zersetzung schmilzt;

unter Verwendung von 98,50 g (0,168 Mol) [2-(N-Aethoxycarbonyl-3-(trifluormethyl)-phenylamino)-phenylazo]-(2-chloracetamido)-malonsäure-diäthylester die rohe 1-[2-[N-Aethoxycarbonyl-3-(trifluormethyl)-phenylamino]-phenyl]-5-(chlormethyl)-1H-1,2,4-triazol-3-carbonsäure, die ab 88° sintert und bei 100-108° unter Zersetzung schmilzt;

unter Verwendung von 91,10 g (0,166 Mol) [2-(N-Aethoxycarbonyl-2,3-dimethylphenylamino)-phenylazo]-(2-chloracetamido)-malonsäurediäthylester die rohe 1-[2-(N-Aethoxycarbonyl-2,3-dimethylphenylamino)-phenyl]-5-(chlormethyl)-1H-1,2,4-triazol-3-carbonsäure, die bei 188-190° unter Zersetzung schmilzt.

Unter Verwendung von 53,70 g (0,097 Mol) [2-(N-Aethoxycarbonylphenylamino)-4-chlorphenylazo]-(2-chloracetamido)-malonsäure-diäthylester die rohe 1-[2-(N-Aethoxycarbonyl-phenylamino)-4-chlorphenyl]-5-(chlormethyl)-1H-1,2,4-triazol-3-carbonsäure, die bei 105° sintert und bei 115-117° unter Zersetzung schmilzt;

unter Verwendung von 59,53 g (0,0957 Mol) rohem [2-(N-Aethoxycarbonyl-2,4-dichlorphenylamino)-5-chlor-phenylazo]-(2-chloracetamido)-malonsäure-diäthylester die rohe 1-[2-(N-Aethoxycarbonyl-2,4-dichlorphenylamino)-5-chlorphenyl]-5-(chlormethyl)-1H-1,2,4-triazol-3-carbonsäure, die bei 186° unter Zersetzung schmilzt.

e) Analog Beispiel 1e) erhält man unter Verwendung von 50,00 g (0,115 Mol) roher 1-[2-(N-Aethoxycarbonyl-4-chlorphenylamino)-phenyl]-5-(chlormethyl)-1H-1,2,4-triazol-3-carbonsäure die rohe 5-(4-Chlorphenyl)-4,5-dihydro-[1,2,4]-triazolo[1,5-a]chinoxalin-2-carbonsäure, die bei 191° sintert und ab 196° unter Zersetzung schmilzt;

unter Verwendung von 77,50 g (0,165 Mol) roher 1-[2-[N-Aethoxycarbonyl-3-(trifluormethyl)-phenylamino]-phenyl]-5-(chlormethyl)-1H-1,2,4-triazol-3-carbonsäure die rohe 5-[3-(Trifluormethyl)-phenyl]-4,5-dihydro-[1,2,4]-triazolo[1,5-a]chinoxalin-2-carbonsäure, die bei 204-206° unter Zersetzung schmilzt;

unter Verwendung von 69,70 g (0,163 Mol) roher 1-[2-(N-Aethoxycarbonyl-2,3-dimethylphenylamino)-phenyl]-5-(chlormethyl-1H-1,2,4-triazol-3-carbonsäure die rohe 5-(2,3-Dimethylphenyl)-4,5-dihydro-[1,2,4]-triazolo[1,5-a]chinoxalin-2-carbonsäure, die bei 178° unter Rotfärbung sintert und bei 195-197° unter Zersetzung schmilzt;

unter Verwendung von 42,20 g (0,097 Mol) roher 1-[2-(N-Aethoxycarbonyl-phenylamino)-4-chlorphenyl]-5-(chlormethyl)-1H-1,2,4-triazol-3-carbonsäure die rohe 7-Chlor-5-phenyl-4,5-dihydro-[1,2,4]-triazolo-[1,5-a]chinoxalin-2-carbonsäure, die bei 178° unter Braunfärbung sintert und bei 205-210° unter Zersetzung schmilzt;

unter Verwendung von 33,30 g (0,066 Mol) roher 1-[2-(N-Aethoxycarbonyl-2,4-dichlorphenylamino)-5-chlorphenyl]-5-(chlormethyl)-1H-1,2,4-triazol-3-carbonsäure die rohe 8-Chlor-5-(2,4-dichlorphenyl)-4,5-dihydro-[1,2,4]-triazolo[1,5-a]chinoxalin-2-carbonsäure, die unter Zersetzung bei 218-222° schmilzt.

f) Analog Beispiel 1f) erhält man unter Verwendung von 43,40 g (0,133 Mol) roher (5-(4-Chlorphenyl)-4,5-dihydro-[1,2,4]-triazolo-[1,5-a]chinoxalin-2-carbonsäure den 5-(4-Chlorphenyl)-4,5-dihydro-[1,2,4]-triazolo[1,5-a]chinoxalin-2-carbonsäure-methylester, der nach Umkristallisation aus Methylenchlorid/Isopropanol bei 143° sintert und von 155° - 212° unter allmählicher Zersetzung schmilzt;

unter Verwendung von 54,10 g (0,150 Mol) roher 5-[3-(Trifluormethyl)-phenyl]-4,5-dihydro-[1,2,4]-triazolo[1,5-a]chinoxalin-2-carbonsäure den 5-[3-(Trifluormethyl)-phenyl]-4,5-dihydro-[1,2,4]-triazolo-[1,5-a]chinoxalin-2-carbonsäure-methylester, der nach Umkristallisation aus Methylenchlorid/Methanol bei 204 - 206° schmilzt;

unter Verwendung von 58,10 g (0,181 Mol) roher 5-(2,3-Dimethylphenyl)-4,5-dihydro- [1,2,4]-triazolo[1,5-a]chinoxalin-2-carbonsäure den 5-(2,3-Dimethylphenyl)-4,5-dihydro- [1,2,4]-triazolo[1,5-a]chinoxalin-2-carbonsäure-methylester, der nach Umkristallisation aus Methylenchlorid/Methanol bei 170-172° schmilzt;

unter Verwendung von 26,60 g (0,0815 Mol) roher 7-Chlor-5-phenyl-4,5-dihydro-[1,2,4]-triazolo[1,5-a]chinoxalin-2-carbonsäure den 7-Chlor-5-phenyl-4,5-dihydro-[1,2,4]-triazolo[1,5-a]chinoxalin-2-carbonsäure-methylester, der nach Umkristallisation aus Methylenchlorid/Isopropanol bei 165° sintert und bei 190-194° schmilzt;

unter Verwendung von 25,40 g (0,0642 Mol) roher 8-Chlor-5-(2,4-dichlorphenyl)-4,5-dihydro-[1,2,4]-triazolo[1,5-a]chinoxalin-2-carbonsäure den 8-Chlor-5-(2,4-dichlorphenyl)-4,5-dihydro-[1,2,4]-triazolo[1,5-a]-chinoxalin-2-carbonsäure-methylester, der nach Umkristallisation aus Methylenchlorid/Isopropanol bei 264-266° schmilzt.

g) Analog Beispiel 1 g) erhält man unter Verwendung von 38,50 g (0,113 Mol) 5-(4-Chlorphenyl)-4,5-dihydro-[1,2,4]-triazolo[1,5-a]-chinoxalin-2-carbonsäure-methylester das 5-(4-Chlorphenyl)-4,5-dihydro-[1,2,4]-triazolo[1,5-a]chinoxalin-2-methanol, das nach Umkristallisation aus Methylenchlorid/Isopropanol bei 190-195° schmilzt;

unter Verwendung von 26,65 g (0,0712 Mol) 5-[3-Trifluormethyl)-phenyl]-4,5-dihydro-[1,2,4]-triazolo[1,5-a]chinoxalin-2-carbonsäure-methylester das 5-[3-(Trifluormethyl)-phenyl]-4,5-dihydro-[1,2,4]-triazolo-[1,5-a]chinoxalin-2-methanol, das nach Umkristallisation aus Methylenchlorid/Isopropanol bei 168-171° schmilzt.

unter Verwendung von 39,84 g (0,119 Mol) 5-(2,3-Dimethylphenyl)-4,5-dihydro-[1,2,4]-triazolo[1,5-a]chinoxalin-2-carbonsäure-methylester das 5-(2,3-Dimethylphenyl)-4,5-dihydro-[1,2,4]-triazolo[1,5-a]-chinoxalin-2-methanol, das nach Umkristallisation aus Methylenchlorid/Isopropanol bei 165° sintert und bei 186-194° schmilzt;

unter Verwendung von 20,80 (0,061 Mol) 7-Chlor-5-phenyl-4,5-dihydro-[1,2,4]-triazolo[1,5-a]chinoxalin-2-carbonsäure-methylester das 7-Chlor-5-phenyl-4,5-dihydro-[1,2,4]-triazolo[1,5-a]chinoxalin-2-methanol, das nach Umkristallisation aus Methylenchlorid/Isopropanol bei 191° sintert und bei 204-207° schmilzt;

unter Verwendung von 19,80 g (0,0483 Mol) 8-Chlor-5-(2,4-dichlorphenyl)-4,5-dihydro-[1,2,4]-triazolo[1,5-a]chinoxalin-2-carbonsäure-methylester das 8-Chlor-5-(2,4-dichlorphenyl)-4,5-dihydro-[1,2,4]-triazolo[1,5-a]chinoxalin-2-methanol vom Smp. 229-231°.

Die beiden in der Literatur nicht beschriebenen, für die Stufe a) benötigten substituierten 2-Nitro-N-phenyl-aniline werden wie folgt hergestellt:

h) Eine Lösung von 80,00 g (0,567 Mol) 1-Fluor-2-nitrobenzol und 182,72 g (1,133 Mol) 3-(Trifluormethyl)-anilin in 400 ml Dimethylsulfoxid wird 68 Stunden bei 130° gerührt. Nach dem Abkühlen giesst man das Reaktionsgemisch auf 1000 ml Wasser und extrahiert zweimal mit 500 ml Aethylacetat. Die vereinigten organischen Extrakte werden 5-mal mit 1-n. Salzsäure, 5-mal mit Wasser und einmal mit gesättigter Natriumchloridlösung gewaschen, über Natriumsulfat getrocknet und im Vakuum eingedampft. Den öligen Rückstand löst man in 200 ml Toluol und filtriert die Lösung durch einen Einlaufbecher, gefüllt mit 610 g Kieselgel, und wäscht die Füllung mit 1000 ml Toluol nach. Das Filtrat wird im Vakuum zur Trockne eingedampft und der Rückstand aus

Methylenchlorid/Hexan umkristallisiert. Nach dem Trocknen im Vakuum schmilzt das erhaltene 2-Nitro-N-[(3-trifluormethyl)-phenyl)]-anilin bei 69-70°.

Auf ganz analoge Weise erhält man ausgehend von 117,00 g (0,830 Mol) 1-Fluor2-nitrobenzol und 222,00 g (1,826 Mol) 2,3-Dimethylanilin in 585 ml Dimethylsulfoxid das N-(2,3-Dimethylphenyl)-2-nitroanilin vom Smp. 118-120° nach Umkristallisation aus Methylenchlorid/Hexan.

Beispiel 6: Zu der Suspension von 10,00 g (0,0358 Mol) 5-Phenyl-4,5-dihydro-pyrido[2,3-e][1,2,4]triazolo[1,5-a]pyrazin-2-methanol in 350 ml abs. Methylenchlorid und 7,24 g (0,072 Mol) Triäthylamin werden bei -8 bis -5° 6,15 g (0,054 Mol) Methansulfonsäurechlorid innerhalb 10 Minuten zugetropft. Die klare, gelbe Reaktionslösung wird 90 Minuten bei Raumtemperatur ausgerührt und hierauf dreimal mit Eiswasser und einmal mit gesättigter Natriumchloridlösung gewaschen. Man trocknet die organische Phase über Natriumsulfat und dampft sie im Vakuum zur Trockene ein.

Der Methansulfonsäureester des 5-Phenyl-4,5-dihydro-pyrido[2,3-e]-[1,2,4]triazolo[1,5-a]pyrazin-2-methanols wird als hellgelber kristalliner Rückstand erhalten und ohne weitere Reinigung sofort weiter umgesetzt, indem man ein eiskaltes Gemisch von 35,4 ml (0,26 Mol) 33%-iger äthanolischer Dimethylaminlösung und 35 ml Aethanol zugibt und das Reaktionsgemisch verschlossen während 3 Stunden rührt. Hierauf dampft man es im Vakuum ein und löst den Rückstand in Aethylacetat und 1-n. Kaliumbicarbonatlösung. Die wässrige Phase wird abgetrennt und noch zweimal mit Aethylacetat extrahiert. Die vereinigten Extrakte werden zweimal mit Wasser und einmal mit gesättigter Natriumchloridlösung gewaschen, über Natriumsulfat getrocknet und im Vakuum eingedampft. Nach Umkristallisieren des Rückstandes aus Methylenchlorid/Isopropanol erhält man das 2-[(Dimethylamino)-methyl]-5-phenyl-4,5-dihydro-pyrido[2,3-e][1,2,4]triazolo[1,5-a]pyrazin, Smp. 135-139°, entsprechend der Formel

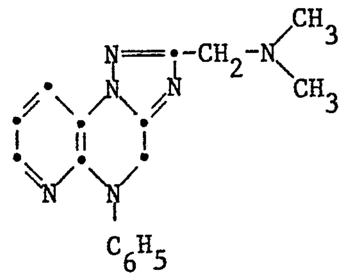

Das als Ausgangsstoff benötigte 5-Phenyl-4,5-dihydro-pyrido[2,3-e]-[1,2,4]triazolo[1,5-a]pyrazin-2-methanol wird wie folgt hergestellt:

a) Eine Lösung von 56,80 g (0,441 Mol) 3-Amino-2-chlor-pyridin (purum) in 440 ml Eisessig und 110 ml konz. Salzsäure wird bei 0-5° mit 88,3 ml (0,441 Mol) einer 5-molaren Natriumnitritlösung diazotiert. Die erhaltene, noch kalte Diazoniumsalzlösung tropft man innerhalb 20 Minuten unter gutem Rühren zu einer Lösung von 88,50 g (0,353 Mol) (2-Chloracetamido)-malonsäurediäthylester [vgl. Ajay Kumar Bose, J. Indian Chem. Soc. 31, 108-110 (1954)] in 1760 ml Methanol, wobei man gleichzeitig aus einem zweiten Tropftrichter 2400 ml gesättigte Kaliumbicarbonatlösung unter schwachem Kühlen so rasch zutropft, dass der pH-Wert des Reaktionsgemisches immer etwa 6 beträgt, und die Reaktionstemperatur zwischen 20° und 23° bleibt. Anschliessend tropft man noch 400 ml gesättigte Kaliumbicarbonatlösung zu (pH-Wert erreicht 7), rührt eine Stunde bei 20° und dann 2 Stunden bei 0-5° aus. Die gebildeten hellgelben Kristalle werden abgenutscht und mit Wasser, wenig Aether und zum Schluss mit Hexan gewaschen. Nach dem Trocknen an der Luft schmilzt der erhaltene (2-Chloracetamido)-(2-chlor-3-pyridylazo)-malonsäurediäthylester, der wechselnde Mengen Kristallwasser enthält, bei 60-72°.

b) Zu 817 ml (0,817 Mol) einer auf 0° vorgekühlten 1-n. Natriumhydroxidlösung gibt man unter gutem Rühren innerhalb 5 Minuten eine auf 2° gekühlte Lösung von 118,30 g (ca. 0,27 Mol) der nach a) hergestellten rohen Azoverbindung in 1060 ml Methanol. Unter Erwärmung auf 18 bis 20° entsteht eine klare rotbraune Lösung, die eine Stunde bei Raumtemperatur ausgerührt wird. Hierauf fügt man 10 g Aktivkohle zu, rührt 5 Minuten bei Raumtemperatur und filtriert das Gemisch über Diatomeenerde unter Nachwaschen mit wenig Methanol/Wasser (1:1). Das klare, gelbe Filtrat wird unter Rühren so lange mit 2-n. Salzsäure versetzt, bis der pH-Wert 3 erreicht ist. Nach dreistündigem Rühren im Eisbad wird die auskristallisierte Säure abgenutscht und gut mit Wasser und Hexan gewaschen. Nach dem Trocknen über Calciumchlorid im Vakuum schmilzt die erhaltene 1-(2-Chlor-3-pyridyl)-5-(chlormethyl)-1H-1,2,4-triazol-3-carbonsäure bei 203-205° unter Zersetzung.

c) Ein Gemisch von 50,00 g (0,183 Mol) der nach b) hergestellten Triazolcarbonsäure, 59,60 g (0,642 Mol) Anilin und 0,05 g Kaliumjodid in 1000 ml Aethanol wird 24 Stunden unter Rückfluss gekocht. Hierauf dampft man das Reaktionsgemisch im Vakuum zur Trockene ein und löst den Rückstand in 200 ml Aether und 500 ml 1-n. Natriumhydroxidlösung. Die alkalische wässrige Phase wird abgetrennt, dreimal mit je 150 ml Aether gewaschen, und hernach durch Zugabe von konz. Salzsäure kongosauer gestellt. Man gibt noch 150 ml Methylenchlorid zu, rührt eine Stunde bei 0° und filtriert die gebildete Carbonsäure ab. Nach dem Waschen mit Wasser und Methylenchlorid trocknet man das Produkt im Vakuum über Calciumchlorid. Die so erhaltene 5-Phenyl-4,5-dihydropyrido[2,3-b][1,2,4]triazolo[1,5-a]pyrazin-2-carbonsäure schmilzt bei 208-213° unter Zersetzung.

Durch Eindampfen der Methylenchlorid-Phasen der Mutterlauge erhält man eine weitere Menge derselben Carbonsäure.

d) Eine Suspension von 49,20 g (0,168 Mol) der nach d) hergestellten Carbonsäure in 490 ml Methanol und 100 ml 6-n. methanolischer Salzsäurelösung wird 16 Stunden unter Rückfluss gekocht, wobei eine klare gelbe Lösung entsteht. Hierauf dampft man im Vakuum ein und löst den Rückstand in Methylenchlorid und eiskalter 1-n. Kaliumbicarbonatlösung. Die abgetrennte organische Phase wird einmal mit kalter 1-n. Kaliumbicarbonatlösung und einmal mit gesättigter Natriumchloridlösung gewaschen, über Natriumsulfat getrocknet und im Vakuum eingedampft. Nach Umkristallisieren des Rückstandes aus Methylenchlorid/Isopropanol erhält man den 5-Phenyl-4,5-dihydro-pyrido[2,3-e]-[ 1,2,4]triazolo[1,5-a]pyrazin-2-carbonsäure-methylester vom Smp. 198-201°.

e) Eine Suspension von 38,50 g (0,125 Mol) des nach e) hergestellten Esters in 1250 ml Methanol und 1250 ml Tetrahydrofuran wird unter Rühren bei Raumtemperatur mit 23,6 g (0,625 Mol) Natriumborhydrid, verteilt auf drei Portionen, versetzt. Unter starkem Schäumen steigt die Temperatur des Reaktionsgemisches dabei auf 33 bis 35° an und wird durch schwaches Kühlen dort gehalten. Anschliessend rührt man 90 Minuten bei Raumtemperatur und nach Zusatz von 1250 ml Eiswasser noch 2 Stunden bei 0-5°. Das ausgefallene Produkt wird abgesaugt und mit Wasser bis pH 6 und Aether gewaschen. Nach dem Trocknen über Calciumchlorid im Vakuum schmilzt das erhaltene 5-Phenyl-4,5-dihydro-pyrido-[2,3-e][1,2,4]triazolo[1,5-a]pyrazin-2-methanol bei 233-237°.

Beispiel 7: Den aus 10,00 g (0,036 Mol) 5-Phenyl-4,5-dihydro-pyrido-[2,3-e][1,2,4]triazolo[1,5-a]pyrazin-2-methanol und 6.15 g (0,054 Mol) Methansulfonsäurechlorid gemäss Beispiel 6 bereiteten rohen Methansulfonsäureester rührt man nach Zugabe von 88 ml (0,934 Mol) 33%-iger äthanolischer Methylaminlösung und 20 ml Aethanol über Nacht bei Raumtemperatur. Hierauf dampft man das Reaktionsgemisch im Vakuum ein und löst den festen Rückstand in Aethylacetat und 1-n. Kaliumbicarbonatlösung. Die wässrige Phase wird abgetrennt und noch einmal mit Aethylacetat extrahiert. Die vereinigten Extrakte werden zweimal mit Wasser und einmal mit gesättigter Natriumchloridlösung gewaschen, über Natriumsulfat getrocknet und im Vakuum eingedampft. Der Rückstand wird in 200 ml Aethanol gelöst und mit einer gesättigten äthanolischen Fumarsäurelösung versetzt, bis der pH-Wert einer mit Wasser versetzten Probe bei 4 liegt. Nach 5 Stunden werden die ausgefallenen Kristalle abfiltriert und zweimal aus Aethanol umkristallisiert.

Nach dem Trocknen schmilzt das erhaltene 2-[(Methylamino)-methyl]-5-phenyl-4,5-dihydro-pyrido[2,3-e][1,2,4]triazolo[1,5-a]pyrazin-fumarat-(1:1) bei 180-183° unter Zersetzung.

Analog erhält man ausgehend von 5,00 g (0,018 Mol) 5-Phenyl-4,5-dihydro-pyrido[2,3-e][1,2,4]triazolo[1,5-a]pyrazin-2-methanol über dessen rohen Methansulfonsäureester unter Verwendung von 12,70 g (0,179 Mol) Pyrrolidin das 2-[(1-Pyrrolidinyl)-methyl]-5-phenyl-4,5-dihydro-pyrido[2,3-e][1,2,4]triazolo[1,5-a]pyrazin vom Smp. 110-113° nach Umkristallisation aus Methylenchlorid/Isopropanol.

Beispiel 8: Analog Beispiel 6 erhält man ausgehend von 12,20 g (0,039 Mol) 5-(2-Chlorphenyl)-4,5-dihydro-pyrido[2,3-e][1,2,4]-triazolo[1,5-a]pyrazin-2-methanol über dessen rohen Methansulfonsäureester unter Verwendung von 53 ml (0,390 Mol) 33%-iger äthanolischer Dimethylaminlösung das 2-[(Dimethylamino)-methyl]-5-(2-chlorphenyl)-4,5-dihydro-pyrido[2,3-e][1,2,4]triazolo[1,5-a]pyrazin, dessen in Aethylacetat bereitetes Hydrochlorid nach Umkristallisation aus Isopropanol bei 224-228° unter Zersetzung schmilzt;

ausgehend von 3,46 g (0,011 Mol) 5-(3-Chlorphenyl)-4,5-dihydro-pyrido-[2,3-e][1,2,4]triazolo[1,5-a]pyrazin-2-methanol über dessen Methansulfonsäureester unter Verwendung von 11 ml (0,08 Mol) 33%-iger äthanolischer Dimethylaminlösung das 2-[(Dimethylamino)-methyl]-5-(3-chlorphenyl)-4,5-dihydro-pyrido[2,3-e] [1,2,4]triazolo[1,5-a]pyrazin vom Smp. 97-100° nach Kristallisation aus Methylenchlorid/Isopropanol;

ausgehend von 6,85 g (0,022 Mol) 5-(4-Chlorphenyl)-4,5-dihydro-pyrido[2,3-e][1,2,4]triazolo[1,5-a]pyrazin-2-methanol über dessen Methansulfonsäureester unter Verwendung von 39 ml (0,218 Mol) 33%-iger äthanolischer Dimethylaminlösung das 2-[(Dimethylamino)-methyl]-5-(4-chlorphenyl)-4,5-dihydro-pyrido[2,3-e][1,2,4]triazolo[1,5-a]-pyrazin vom Schmelzpunkt 185-188° nach Kristallisation aus Methylenchlorid/Isopropanol;

ausgehend von 6,30 g (0,020 Mol) 5-(4-Methoxy-phenyl)-4,5-dihydro-pyrido[2,3-e][1,2,4]triazolo[1,5-a]pyrazin-2-methanol über dessen rohen Methansulfonsäureester unter Verwendung von 21 ml (0,12 Mol) 33%-iger äthanolischer Dimethylaminlösung das 2-[(Dimethylamino)-methyl]-5-(4-methoxy phenyl)-4,5-dihydro-pyrido[2,3-e][1,2,4]triazolo[1,5-a]pyrazin, dessen in Aethylacetat bereitetes Hydrochlorid nach Umkristallisation aus Isopropanol bei 215-222° unter Zersetzung schmilzt, und

ausgehend von 20,20 g (0,071 Mol) 5-Cyclohexyl-4,5-dihydro-pyrido-[2,3-e][1,2,4]triazolo[1,5-a]pyrazin-2-methanol über dessen rohen Methansulfonsäuremethylester unter Verwendung von 97 ml (0,71 Mol) 33%-iger äthanolischer Dimethylaminlösung das 2-[(Dimethylamino)-methyl]-5-cyclohexyl-4,5-dihydro-pyrido[2,3-e][1,2,4]triazolo[1,5-a]-pyrazin, dessen in Aethylacetat bereitetes Hydrochlorid nach Umkristallisation aus Methylenchlorid/Isopropanol bei 278-281° unter Zersetzung schmilzt.

Die benötigten Ausgangsstoffe werden wie folgt hergestellt:

a) Analog Beispiel 6c und 6d erhält man unter Verwendung von 31,40 g (0,115 Mol) 1-(2-Chlor-3-pyridyl)-5-(chlormethyl)-1H-1,2,4-triazol-3-carbonsäure und 206,00 g (1,61 Mol) 2-Chloranilin die 5-(2-Chlorphenyl)-4,5-dihydro-pyrido[2,3-e][1,2,4]triazolo[1,5-a]pyrazin-2-carbonsäure und deren Methylester, der nach Umkristallisation aus Methylenchlorid/Isopropanol bei 155-163° schmilzt;

unter Verwendung von 7,36 g (0,027 Mol) 1-(2-Chlor-3-pyridyl)-5-(chlormethyl)-1H-1,,2,4-triazol-3-carbonsäure und 48,10 g (0,378 Mol) 3-Chloranilin die 5-(3-Chlorphenyl)-4,5-dihydro-pyrido[2,3e][1,2,4]triazolo[1,5-a]pyrazin-3-carbonsäure und deren Methylester, der nach Umkristallisation aus Methylenchlorid/Isopropanol bei 213-216° schmilzt;

unter Verwendung von 8,91 g (0,033 Mol) 1-(2-Chlor-3-pyridyl)-5-(chlormethyl)-1H-1,2,4-triazol-3-carbonsäure und 20,90 g (0,164 Mol) p-Chloranilin die 5-(4-Chlorphenyl)-4,5-dihydro-pyrido[2,3-e][1,2,4]triazolo-[1,5-a]pyrazin-2-carbonsäure und deren Methylester, der nach Umkristallisation aus Methylenchlorid/Aethylacetat bei 234-237° unter Zersetzung schmilzt;

unter Verwendung von 10,00 g (0,037 Mol) 1-(2-Chlor-3-pyridyl)-5-(chlormethyl)-1H-1,2,4-triazol-3-carbonsäure und 15,80 g (0,128 Mol) 4-Methoxy-anilin die 5-(4-Methoxy-phenyl)-4,5-dihydro-pyrido[2,3-e]-[1,2,4]triazolo[1,5-a]pyrazin-2-carbonsäure und deren Methylester, der nach Umkristallisation aus Methylenchlorid/Isopropanol bei 190-196° schmilzt;

unter Verwendung von 21,80 g (0,080 Mol) 1-(2-Chlor-3-pyridyl)-5-(chlormethyl)-1H-1,2,4-triazol-3-carbonsäure und 35,70 g (0,360 Mol) Cyclohexylamin die 5-Cyclohexyl-4,5-dihydro-pyrido[2,3-e][1,2,4]triazolo[1,5-a]pyrazin-2-carbonsäure und deren Methylester,der nach Umkristallisation aus Methylenchlorid/Isopropanol bei 170-176° schmilzt.

b) Analog Beispiel 6e erhält man unter Verwendung von 15,00 g (0,044 Mol) 5-(2-Chlorphenyl)-4,5-dihydro-pyrido[2,3-e][1,2,4]triazolo[1,5-a]-pyrazin-2-carbonsäure-methylester und 8,28 g (0,219 Mol) Natriumborhydrid das 5-(2-Chlorphenyl)-4,5-dihydro-pyrido[2,3-e][1,2,4]triazolo-[1,5-a]pyrazin-2-methanol vom Smp. 193-196°;

unter Verwendung von 3,80 g (0,011 Mol) 5-(3-Chlorphenyl)-4,5-dihydro-pyrido[2,3-e][1,2,4]triazolo[1,5-a]pyrazin-2-carbonsäure-methylester und 4,20 g (0,112 Mol) Natriumborhydrid das 5-(3-Chlorphenyl)-4,5-dihydro-pyrido[2,3-e][1,2,4]triazolo[1,5-a]pyrazin-2-methanol vom Smp. 204-206°;

unter Verwendung von 7,90 g (0,023 Mol) 5-(4-Chlorphenyl)-4,5-dihydro-pyrido[2,3-e][1,2,4]triazolo[1,5-a]pyrazin-2-carbonsäure-methylester und 4,37 g (0,116 Mol) Natriumborhydrid das 5-(4-Chlorphenyl)-4,5-dihydro-pyrido[2,3-e][1,2,4]triazolo[1,5-a]pyrazin-2-methanol vom Smp. 240-245° unter Zersetzung;

unter Verwendung von 8,10 g (0,024 Mol)5-(4-Methoxy-phenyl)-4,5-dihydro-pyrido[2,3-e][1,2,4]triazolo[1,5-a]pyrazin-2-carbonsäure-methylester und 4,50 g (0,120 Mol) Natriumborhydrid das 5-(4-Methoxy-phenyl)-4,5-dihydro-pyrido[2,3-e][1,2,4]triazolo[1,5-a]pyrazin-2-methanol vom Smp. 226 - 230°, und

unter Verwendung von 29,80 g (0,095 Mol) 5-Cyclohexyl-4,5-dihydro-pyrido[2,3-e][1,2,4]triazolo[1,5-a]pyrazin-2-carbonsäure-methylester und 18,00 g (0,476 Mol) Natriumborhydrid nach Eindampfen des Reaktionsgemisches und extraktiver Aufarbeitung mit Methylenchlorid das 5-Cyclohexyl-4,5-dihydro-pyrido[2,3-e][1,2,4]triazolo[1,5-a]pyrazin-2-methanol, das nach Umkristallisation aus Methylenchlorid/Isopropanol bei 190 - 194° schmilzt.

Beispiel 9: Zu der Lösung von 8,00 g (0,027 Mol) 4-Methyl-5-phenyl-4,5-dihydro-[1,2,4]-triazolo[1,5-a]chinoxalin-2-methanol in 270 ml abs. Methylenchlorid und 5,50 g (0,055 Mol) Triäthylamin werden unter Stickstoff bei 0° 4,70 g (0,041 Mol) Methansulfonsäurechlorid innerhalb 5 Minuten zugetropft. Nach einstündigem Rühren bei 0 bis 4° giesst man das klare Reaktionsgemisch auf Eis-Wasser-Gemisch, trennt die organische Phase ab und extrahiert die wässrige Phase noch einmal mit Methylenchlorid. Die Extrakte werden zweimal mit eiskaltem Wasser und einmal mit gesättigter Natriumchloridlösung gewaschen, über Natriumsulfat getrocknet und im Vakuum zur Trockne eingedampft.

Der als Rückstand von honigartiger Konsistenz erhaltene Methansulfonsäureester des 4-Methyl-5-phenyl-4,5-dihydro-[1,2,4]-triazolo[1,5-a]chinoxalin-2-methanols
wird ohne weitere Reinigung umgesetzt, indem man ein kaltes Gemisch von 37,5 ml (0,274 Mol) 33 %iger äthanolischer Dimethylaminlösung und 37,5 ml Aethanol zugibt und das Reaktionsgemisch verschlossen während einer Stunde stehen lässt. Hierauf dampft man es im Vakuum ein und löst den Rückstand in Aethylacetat und 1-n. Kaliumbicarbonatlösung. Die wässrige Phase wird abgetrennt und noch einmal mit Aethylacetat extrahiert. Die vereinigten organischen Extrakte werden dreimal mit Wasser und einmal mit gesättigter Natriumchloridlösung gewaschen und über Natriumsulfat getrocknet. Nach Abfiltrieren des Trocknungsmittels versetzt man das Filtrat unter gutem Rühren tropfenweise mit ätherischer Chlorwasserstofflösung, bis der pH-Wert 3 erreicht ist. Nach 5 Stunden Stehen bei Raumtemperatur wird das gebildete rohe Hydrochlorid abfiltriert und mit Aethylacetat gewaschen. Hierauf wird es in 50 ml Methylenchlorid und 100 ml Aethylacetat gelöst. Nach dem Abdampfen des Methylenchlorids im Vakuum bei 40° kristallisiert das gewünschte Hydrochlorid wieder aus. Man lässt das Gemisch 2 Stunden im Eisschrank stehen, filtriert dann die Kristalle ab und wäscht sie mit Aethylacetat. Nach dem Trocknen im Hochvakuum bei 120° schmilzt das erhaltene 2-[(Dimethylamino)-methyl]-4-methyl-5-phenyl-4,5-dihydro-[1,2,4]-triazolo[1,5-a]chinoxalin-hydrochlorid bei 195 - 198°.

Das als Ausgangsstoff benötigte 4-Methyl-5-phenyl-4,5-dihydro-[1,2,4]-triazolo[1,5-a]chinoxalin-2-methanol wird wie folgt hergestellt:

a) Zu der Suspension von 50,0 g (0,272 Mol) Aminomalonsäure-dimethylester-hydrochlorid in 250 ml abs. Methylenchlorid werden innerhalb einer Stunde 30,0 ml (0,30 Mol) 2-Chlorpropionsäurechlorid zugetropft. Nach 4-stündigem Kochen unter Rückfluss ist die Chlorwasserstoffentwicklung beendet. Man kühlt die Suspension auf Raumtemperatur ab, gibt 100 ml eiskaltes Wasser zu, trennt die organische Phase

ab und wäscht sie dreimal mit Wasser und einmal mit gesättigter Natriumchloridlösung. Nach dem Eindampfen im Vakuum löst man den Rückstand in 100 ml Methylenchlorid, gibt langsam 400 ml Hexan zu und lässt das Gemisch 2 Stunden bei Raumtemperatur stehen. Hierauf werden die entstandenen Kristalle abfiltriert und mit Methylenchlorid-Hexan (1:4) gewaschen. Nach dem Trocknen im Vakuum schmilzt der erhaltene (2-Chlor-propionamido)-malonsäure-dimethylester bei 100 bis 102°.

b) Eine Lösung von 26,20 g (0,102 Mol) des gemäss Beispiel 1b) bereiteten (2-Aminophenyl)-phenyl-carbaminsäure-äthylesters in 246 ml Eisessig und 62 ml konz. Salzsäure wird bei 0 - 5°mit 20,4 ml (0,102 Mol) einer 5-molaren Natriumnitritlösung innerhalb 20 Minuten diazotiert. Die erhaltene Diazoniumsalzlösung versetzt man mit 60 g Eis und lässt 24,23 g (0,102 Mol) (2-Chlor-propionamido)-malonsäure-dimethylester in 243 ml Aceton rasch zutropfen. Anschliessend tropft man bei 0 - 5° im Laufe von 30 Minuten 500 ml einer gesättigten Kaliumcarbonatlösung zu, wobei der pH-Wert am Schluss 6 erreicht. Nach einstündigem Ausrühren bei 0 bis 5° gibt man 300 ml Aethylacetat zu, trennt die organische Phase ab und extrahiert die wässrige Phase noch einmal mit Aethylacetat. Die vereinigten organischen Extrakte werden viermal mit Wasser und zweimal mit gesättigter Natriumchloridlösung gewaschen, über Natriumsulfat getrocknet und im Vakuum eingedampft, wobei der orangefarbene [2-(N-Aethoxycarbonyl-phenylamino)-phenylazo]-(2-chlor-propionamido)-malonsäure-dimethylester von honigartiger Konsistenz erhalten wird. Er kann ohne weitere Reinigung zur nächsten Stufe verwendet werden.

c) Zu 316 ml (0,316 Mol) einer auf 0° vorgekühlten 1-n. Natriumhydroxidlösung gibt man unter gutem Rühren innerhalb 3 Minuten eine Lösung von 53,0 g (ca. 0,102 Mol) der nach b) hergestellten Azoverbindung in 530 ml Methanol. Man rührt weitere 90 Minuten bei Raumtemperatur und tropft anschliessend Eisessig zu, bis der pH-Wert 5 erreicht ist.

Die klare Lösung wird mit 2-n. Salzsäure bis zur kongosauren Reaktion versetzt. Anschliessend tropft man in das Reaktionsgemisch innerhalb 3 Stunden 350 ml Wasser zu, wobei allmählich Kristallisation erfolgt. Nach 6-stündigem Rühren bei 0° werden die gebildeten Kristalle abfiltriert und ausgiebig mit Wasser gewaschen. Nach dem Trocknen über Calciumchlorid im Vakuum schmilzt die erhaltene 1-[2-(N-Aethoxycarbonyl-phenylamino)-phenyl]-5-(1-chloräthyl)-1H-[1,2,4]-triazol-3-carbonsäure bei 153 bis 156°.

d) Eine Suspension von 37,90 g (0,091 Mol) der nach c) hergestellten Carbonsäure in 210 ml 48 %iger Bromwasserstoffsäure wird während 12 Stunden bei einer Badtemperatur von 105° unter Stickstoff gerührt, wobei eine klare grüne Lösung entsteht. Anschliessend kühlt man das Reaktionsgemisch auf Raumtemperatur ab und tropft innerhalb 90 Minuten unter Stickstoff 530 ml Wasser zu. Nach 3-stündigem Kühlen auf 0° wird die Carbonsäure abgesaugt, mit total 600 ml Wasser bis pH 4 gewaschen und im Vakuumexsikkator über Calciumchlorid getrocknet. Die erhaltene 4-Methyl-5-phenyl-4,5-dihydro-[1,2,4]-triazolo-[1,5-a]chinoxalin-2-carbonsäure sintert bei 176° und schmilzt ab 181° unter Zersetzung.

e) Eine Suspension von 26,70 g (0,087 Mol) der nach d) hergestellten Carbonsäure in 535 ml Methanol und 106 ml 6-n. methanolischer Salzsäurelösung wird unter Stickstoff 16 Stunden bei Siedetemperatur unter Rückfluss gerührt, wobei rasch eine klare hellgelbe Lösung entsteht. Anschliessend dampft man das Reaktionsgemisch im Vakuum zur Trockne ein. Den Rückstand löst man in Methylenchlorid und Eiswasser, trennt die organische Phase ab und extrahiert die wässrige Phase noch einmal mit Methylenchlorid. Die vereinigten organischen Extrakte werden zweimal mit eiskalter 1-n. Kaliumbicarbonatlösung und zweimal mit gesättigter Natriumchloridlösung gewaschen, über Natriumsulfat getrocknet und im Vakuum eingedampft. Zum Rückstand von honigartiger Konsistenz gibt man 60 ml Aether und hernach 60 ml Hexan. Nach 4 Stunden werden die gebildeten Kristalle abfiltriert und mit Aether-Hexan (1:1) gewaschen. Nach dem Trocknen im Vakuum sintert der erhaltene 4-Methyl-5-phenyl-4,5-dihydro-[1,2,4]-triazolo[1,5-a]-chinoxalin-2-carbonsäuremethylester bei 134° und schmilzt bei 139 bis 141°.

f) Eine Lösung von 16,60 g (0,052 Mol) des nach e) hergestellten Esters in 166 ml Methanol und 166 ml Tetrahydrofuran wird unter Rühren und Stickstoff bei Raumtemperatur innerhalb 10 Minuten mit total 5,48 g (0,145 Mol) Natriumborhydrid, verteilt auf drei Portionen, versetzt. Unter starkem Schäumen steigt die Temperatur des Reaktionsgemisches dabei auf 29° an. Man rührt 3 Stunden bei Raumtemperatur und nach Zusatz von 335 ml Eis-Wasser-Gemisch noch 2 Stunden bei 0°. Der ausgefallene Alkohol wird abgesaugt und mit Wasser bis pH 6 gewaschen. Nach dem Trocknen über Calciumchlorid im Vakuumexsikkator sintert das erhaltene 4-Methyl-5-phenyl-4,5 dihydro-[1,2,4]-triazolo-[1,5-a]chinoxalin-3-methanol bei 150° und schmilzt bei 157 - 160°.

Beispiel 10: Zu der Lösung von 7,70 g (0,022 Mol) N,N,4,4-Tetramethyl-5-phenyl-4,5-dihydro[1,2,4]-triazolo[1,5-a]chinoxalin-2-carboxamid in 1100 ml abs. Toluol tropft man bei 0 bis 2° unter Stickstoff 55,40 ml (0,066 Mol) einer 1,2-molaren Lösung von Diisobutylaluminiumhydrid in Toluol innerhalb 20 Minuten zu.. Das Reaktionsgemisch rührt man noch eine Stunde bei 5°, tropft dann 15 ml Isopropanol und danach 160 ml eiskaltes Wasser und soviel konzentrierte Natronlauge zu,bis der pH-Wert 11 erreicht ist. Man trennt die organische Phase ab und extrahiert die wässrige Phase noch einmal mit Toluol. Die vereinigten organischen Extrakte werden zweimal mit 2-n. Natronlauge, zweimal mit Wasser und einmal mit gesättigter Natriumchloridlösung gewaschen, über Natriumsulfat getrocknet und im Vakuum eingedampft. Den Rückstand von honigartiger Konsistenz löst man in Aethylacetat und chromatographiert die Lösung an einer Säule von 180 g Kieselgel

zunächst mit 2 l Aethylacetat und hernach mit 3 l Methylenchlorid-Methanol (95:5). Die Fraktionen, welche das gewünschte Produkt enthalten, werden im Vakuum eingedampft, in Aethylacetat gelöst und unter Rühren mit ätherischer Chlorwasserstofflösung versetzt, bis der pH-Wert 4 erreicht ist. Nach einstündigem Rühren im Eisbad wird das gebildete reine Hydrochlorid abfiltriert und mit Aethylacetat gewaschen. Nach dem Trocknen im Hochvakuum bei 80° über Kaliumhydroxid schmilzt das erhaltene 4,4-Dimethyl-2-[(dimethylamino)-methyl]-5-phenyl-4,5-dihydro-[1,2-4]-triazolo[1,5-a]chinoxalin-hydrochlorid bei 206 - 207°.

Das als Ausgangsstoff benötigte N,N,4,4-Tetramethyl-5-phenyl-4,5-dihydro-[1,2,4]-triazolo[1,5-a]chinoxalin-2-carboxamid wird wie folgt hergestellt:

a) Eine Lösung von 18,0 g (0,056 Mol) des gemäss Beispiel 9e) hergestellten 4-Methyl-5-phenyl-4,5-dihydro-[1,2,4]-triazolo[1,5-a]chinoxalin-2-carbonsäuremethylesters in 900 ml Methanol und 154 ml (1,12 Mol) 33 %iger äthanolischer Dimethylaminlösung wird während 24 Stunden verschlossen gerührt. Hierauf dampft man das Reaktionsgemisch im Vakuum ein und löst den Rückstand in Methylenchlorid und Eis-Wasser-Gemisch. Die organische Phase wird zweimal mit kalter 5 %iger Kaliumbicarbonatlösung und einmal mit gesättigter Natriumchloridlösung gewaschen, über Natriumsulfat getrocknet und im Vakuum eingedampft. Nach Umkristallisation des Rückstandes aus 100 ml Isopropanol und Trocknen im Vakuumexsikkator über Calciumchlorid schmilzt das erhaltene N,N,4-Trimethyl-5-phenyl-4,5-dihydro-[1,2,4]-triazolo-[1,5-a]chinoxalin-2-carboxamid bei 150 bis 152°.

b) Zu einer auf -70° gekühlten Lösung von 3,60 ml (0,026 Mol) Diisopropylamin in 78 ml abs. Tetrahydrofuran tropft man innerhalb 15 Minuten unter Stickstoff mit der Spritze 16 ml (0,026 Mol) einer

1,6-molaren Lösung von Butyllithium in Hexan. Die klare gelbliche Reaktionslösung wird während 5 Minuten bei -10° gerührt. Nach erneutem Abkühlen auf -70° tropft man innerhalb 30 Minuten eine Lösung von 7,80 g (0,023 Mol) des gemäss Beispiel 10 b) hergestellten Carbonsäureamides in 4,5 ml Hexamethylphosphorsäuretriamid und 31 ml abs. Tetrahydrofuran zu. Die tief braun-rote Lösung wird 15 Minuten bei -70° und nach Zusatz von 3,20 ml (0,051 Mol) Methyljodid weitere 90 Minuten bei -70° gerührt. Dann lässt man die Reaktionslösung sich auf 0° erwärmen, giesst sie auf Eis-Wasser-Gemisch und extrahiert zweimal mit Aethylacetat. Die organischen Extrakte werden zweimal mit eiskalter 2-n. Salzsäure, zweimal 1-n. Kaliumbikarbonatlösung und einmal mit gesättigter Natriumchloridlösung gewaschen, über Natriumsulfat getrocknet und im Vakuum zur Trockne eingedampft. Den Rückstand von honigartiger Konsistenz, der langsam zu kristallisieren beginnt, löst man in Aethylacetat und chromatographiert die Lösung an einer Säule von 200 g Kieselgel mit dem Elutionsmittel Aethylacetat. Die Fraktionen, welche das gewünschte Produkt enthalten, werden vereinigt und aus Isopropanol umkristallisiert. Nach dem Trocknen im Vakuumsexsikkator über Calciumchlorid schmilzt das erhaltene N,N,4,4-Tetramethyl-5-phenyl-4,5-dihydro-[1,2,4]-triazolo[1,5-a]chinoxalin-2-carboxamid bei 147-149°.

Beispiel 11: Tabletten enthaltend 100 mg 2-[(Dimethylamino)-methyl]-5-phenyl-4,5-dihydro-[1,2,4]-triazolo[1,5-a]chinoxalin-hydrochlorid als Wirkstoff können beispielsweise in folgender Zusammensetzung hergestellt werden.

| Zusammensetzung | pro Tablette |
|---|---|
| Wirkstoff | 100 mg |
| Milchzucker | 50 mg |
| Weizenstärke | 73 mg |
| Kolloidale Kieselsäure | 13 mg |
| Talk | 12 mg |
| Magnesiumstearat | 2 mg |
| | 250 mg |

Herstellung Der Wirkstoff wird mit Milchzucker, einem Teil der Weizenstärke und mit kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist. Die Masse wird durch ein Sieb von ca. 3 mm Maschenweise getrieben, getrocknet und das trockene Granulat nochmals durch ein Sieb getrieben. Darauf werden die restliche Weizenstärke, Talk und Magnesiumstearat zugemischt. Die erhaltene Mischung wird zu Tabletten von 250 mg mit Bruchkerbe(n) verpresst.

Beispiel 12: Analog Beispiel 11 können Tabletten mit einem Gehalt von je 50 mg Wirkstoff hergestellt werden unter Verwendung von je 50 mg 2-[(Dimethylamino)-methyl]-5-phenyl-4,5-dihydro-[1,2,4]-triazolo-[1,5-a]chinoxalin und je 100 mg Milchzucker pro Tablette.

Beispiel 13: Um 1000 Kapseln mit je 100 mg Wirkstoffgehalt herzustellen, mischt man 100 g 2-[(Dimethylamino)-methyl]-5-phenyl-4,5-dihydro-[1,2,4]-triazolo[1,5-a]chinoxalin-hydrochlorid mit 173,0 g Lactose, befeuchtet die Mischung gleichmässig mit einer wässrigen Lösung von 2,0 g Gelatine und granuliert sie durch ein geeignetes Sieb (z.B. Sieb III nach Ph. Helv. V.). Das Granulat mischt man mit 10,0 g getrockneter Maisstärke und 15,0 g Talk und füllt es gleichmässig in 1000 Hartgelatinekapseln der Grösse 1.

Anstelle von 2-[(Dimethylamino)-methyl]-5-phenyl-4,5-dihydro-[1,2,4]-triazolo[1,5-a]chinoxalin-hydrochlorid kann man in den obigen Beispielen 11, 12 und 13 auch ein pharmazeutisch annehmbares Säureadditionssalz einer andern Verbindung der allgemeinen Formel I verwenden.

Patentansprüche (für alle benannten Länder ausser Oesterreich)

1. Ein polycyclischer Polyazaheterocyclus der allgemeinen Formel I

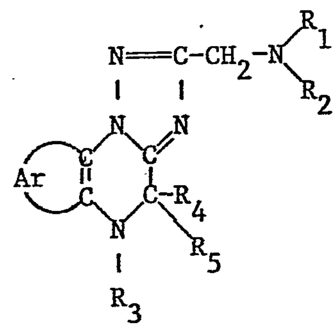

(I)

in welcher $R_1$ und $R_2$ unabhängig voneinander Wasserstoff, Niederalkyl oder Hydroxyniederalkyl, oder zusammen Niederalkylen oder Aethylenoxyäthylen, Aethylenazaäthylen oder N-Niederalkyl- oder N-(2-Hydroxyniederalkyl)-äthylenazaäthylen, $R_3$ Wasserstoff oder einen niederen aliphatischen oder gesättigten, niederen cycloaliphatischen Kohlenwasserstoffrest oder unsubstituiertes oder substituiertes Phenyl, $R_4$ und $R_5$ Wasserstoff oder Niederalkyl und Ar einen unsubstituierten oder substituierten Benzo- oder Pyridorest bedeutet, und dessen Säureadditionssalze.

2. Eine Verbindung der im Anspruch 1 angegebenen allgemeinen Formel I, in der $R_1$ und $R_2$ die im Anspruch 1 angegebene Bedeutung haben, $R_3$ einen niederen aliphatischen oder einen gesättigten niederen cycloaliphatischen Kohlenwasserstoffrest oder unsubstituiertes oder durch Halogen bis Atomnummer 35, Niederalkyl, Niederalkoxy, Niederalkylthio, Trifluormethyl, Cyano oder Nitro substituiertes Phenyl und Ar einen unsubstituierten oder durch Substituenten der vorgenannten Art substituierten Benzo- oder Pyrido[2,3-e]rest bedeutet und $R_4$ und $R_5$ die im Anspruch 1 angegebene Bedeutung haben, und deren Säureadditionssalze.

3. Eine Verbindung der im Anspruch 1 angegebenen allgemeinen Formel I, in der $R_1$ Niederalkyl, $R_2$ Wasserstoff oder Niederalkyl, oder $R_1$ und $R_2$ zusammen Niederalkylen, $R_3$ unsubstituiertes oder durch Halogen bis Atomnummer 35 substituiertes Phenyl oder niederes Cycloalkyl und Ar einen unsubstituierten oder durch Halogen bis Atomnummer 35 substituierten Benzo- oder Pyrido[2,3-e]rest bedeutet und $R_4$ und $R_5$ die im Anspruch 1 angegebene Bedeutung haben, und deren Säureadditionssalze.

4. Eine Verbindung der im Anspruch 1 angegebenen allgemeinen Formel I, in der $R_1$ Niederalkyl, $R_2$ Wasserstoff oder Niederalkyl und $R_1$ und $R_2$ zusammen Niederalkylen,$R_3$ unsubstituiertes oder durch Halogen bis Atomnummer 35 substituiertes Phenyl, und Ar einen unsubstituierten oder durch Halogen bis Atomnummer 35 substituierten Benzorest oder den Pyrido[2,3-e]rest bedeutet, $R_4$ die im Anspruch 1 angegebene Bedeutung hat und $R_5$ Wasserstoff oder Methyl bedeutet, und deren Säureadditionssalze.

5. Eine Verbindung der im Anspruch 1 angegebenen allgemeinen Formel I, in der $R_1$ Methyl, $R_2$ Wasserstoff oder $R_1$ und $R_2$ zusammen Tetramethylen, $R_3$ unsubstituiertes oder durch Chlor substituiertes Phenyl, und Ar einen unsubstituierten oder durch Chlor substituierten Benzorest oder den Pyrido[2,3-e]rest bedeutet, $R_4$ Wasserstoff oder Methyl und $R_5$ Wasserstoff oder Methyl bedeutet, und deren Säureadditionssalze.

6. Eine Verbindung der im Anspruch 1 angegebenen allgemeinen Formel I, in der $R_1$ Methyl, $R_2$ Wasserstoff oder Methyl, $R_3$ Phenyl, $R_4$ Wasserstoff oder Methyl, $R_5$ Wasserstoff oder Methyl und Ar den Benzo- oder Pyrido[2,3-e]rest bedeutet, und deren Säureadditionssalze.

7. 2-[(Dimethylamino)-methyl]-5-phenyl-4,5-dihydro-[1,2,4]-triazolo-[1,5-a]chinoxalin und dessen Säureadditionssalze.

8. 2-[(Methylamino)-methyl]-5-phenyl-4,5-dihydro-[1,2,4]-triazolo-[1,5-a]chinoxalin und dessen Säureadditionssalze.

9. 2-[(Diäthylamino)-methyl]-5-phenyl-4,5-dihydro-[1,2,4]-triazolo-[1,5-a]chinoxalin und dessen Säureadditionssalze.

10. 2-[(Piperidino)-methyl]-5-phenyl-4,5-dihydro-[1,2,4]-triazolo-[1,5-a]chinoxalin und dessen Säureadditionssalze.

11. 2-[(Dimethylamino)-methyl]-5-(4-chlorphenyl)-4,5-dihydro-[1,2,4]-triazolo[1,5-a]chinoxalin und dessen Säureadditionssalze.

12. 2-[(Dimethylamino)-methyl]-5-[3-(trifluormethyl)-phenyl]-4,5-dihydro-[1,2,4]-triazolo[1,5-a]chinoxalin und dessen Säureadditionssalze.

13. 2-[(Dimethylamino)-methyl]-5-(2,3-dimethylphenyl)-4,5-dihydro-[1,2,4]-triazolo[1,5-a]chinoxalin und dessen Säureadditionssalze.

14. 7-Chlor-2-[(dimethylamino)-methyl]-5-phenyl-4,5-dihydro-[1,2,4]-triazolo[1,5-a]chinoxalin und dessen Säureadditionssalze.

15. 8-Chlor-2-[(dimethylamino)-methyl]-5-(2,4-dichlorphenyl)-4,5-dihydro-[1,2,4]-triazolo[1,5-a]chinoxalin und dessen Säureadditionssalze.

16. 2-[(Dimethylamino)-methyl]-4-methyl-5-phenyl-4,5-dihydro-[1,2,4]-triazolo[1,5-a]chinoxalin und dessen Säureadditionssalze.

17. 4,4-Dimethyl-2-[(dimethylamino)-methyl]-5-phenyl-4,5-dihydro-[1,2,4]-triazolo[1,5-a]chinoxalin und dessen Säureadditionssalze.

18. 2-[(Dimethylamino)-methyl]-5-phenyl-4,5-dihydro-pyrido[2,3-e][1,2,4]-triazolo[1,5-a]pyrazin und dessen Säureadditionssalze.

19. 2-[(Methylamino)-methyl]-5-phenyl-4,5-dihydro-pyrido[2,3-e]-[1,2,4]triazolo[1,5-a]pyrazin und dessen Säureadditionssalze.

20. 2-[(1-Pyrrolidinyl)-methyl]-5-phenyl-4,5-dihydro-pyrido[2,3-e]-[1,2,4]triazolo[1,5-a]pyrazin und dessen Säureadditionssalze.

21. Die pharmazeutisch annehmbaren Säureadditionssalze einer Verbindung gemäss einem der Ansprüche 1 bis 6.

22. Die pharmazeutisch annehmbaren Säureadditionssalze einer Verbindung gemäss einem der Ansprüche 7 bis 20.

23. Eine Verbindung gemäss einem der Ansprüche 1 bis 20 und deren pharmazeutisch annehmbare Säureadditionssalze zur Anwendung in einem Verfahren zur prophylaktischen und/oder therapeutischen Behandlung des tierischen oder menschlichen Körpers.

24. Eine Verbindung gemäss einem der Ansprüche 1 bis 20 und deren pharmazeutisch annehmbare Säureadditionssalze als Antidepressiva und Anxiolytica.

25. Pharmazeutische Präparate, gekennzeichnet durch einen Gehalt an einer Verbindung der im Anspruch 1 angegebenen allgemeinen Formel I, in welcher $R_1$, $R_2$, $R_3$, $R_4$, $R_5$ und Ar die im Anspruch 1 angegebene Bedeutung haben, oder an einem pharmazeutisch annehmbaren Säureadditionssalz einer solchen.

26. Pharmazeutische Präparate, gekennzeichnet durch einen Gehalt an einer Verbindung der im Anspruch 1 angegebenen allgemeinen Formel I, in welcher $R_1$ , $R_2$, $R_4$ und $R_5$ die im Anspruch 1 angegebene Bedeutung und $R_3$ und Ar die im Anspruch 2 angegebene Bedeutung haben, oder an

einem pharmazeutisch annehmbaren Säureadditionssalz einer solchen.

27. Pharmazeutische Präparate, gekennzeichnet durch einen Gehalt an einer Verbindung der im Anspruch 1 angegebenen allgemeinen Formel I, in welcher $R_1$, $R_2$, $R_3$, $R_5$ und Ar die im Anspruch 4 angegebene Bedeutung haben und $R_4$ die im Anspruch 1 angegebene Bedeutung hat, oder an einem pharmazeutisch anmehmbaren Säureadditionssalz einer solchen.

28. Pharmazeutische Präparate, gekennzeichnet durch einen Gehalt an 2-[(Dimethylamino)-methyl]-5-phenyl-4,5-dihydro-[1,2,4]-triazolo[1,5-a]-chinoxalin oder an einem pharmazeutisch annehmbaren Säureadditions-salz desselben.

29. Pharmazeutische Präparate nach einem der Ansprüche 25 bis 28, gekennzeichnet durch einen zusätzlichen Gehalt an mindestens einem pharmazeutischen Träger- oder Hilfsstoff.

30. Verfahren zur Herstellung von Verbindungen der im Anspruch 1 angegebenen allgemeinen Formel I und deren Säureadditionssalzen, dadurch gekennzeichnet, dass man

(a) einen reaktionsfähigen Ester einer Verbindung der allgemeinen Formel II

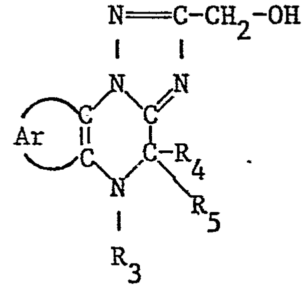

(II) ,

in welcher

$R_3$, $R_4$, $R_5$ und Ar die im Anspruch 1 angegebene Bedeutung haben, mit einer Verbindung der allgemeinen Formel III

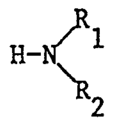 (III) ,

in welcher $R_1$ und $R_2$ die im Anspruch 1 angegebene Bedeutung haben, umsetzt, oder

(b) in einer Verbindung der allgemeinen Formel IV

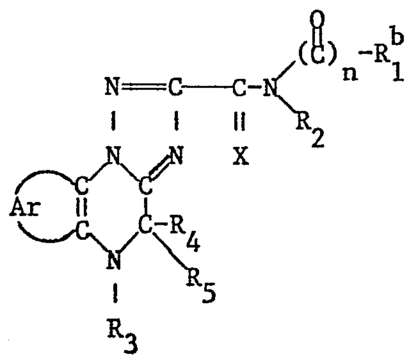 (IV) ,

in welcher n Null oder 1 und X Sauerstoff bedeutet, oder, falls n 1 bedeutet, auch zwei Wasserstoffatome bedeuten kann, $R_1^b$ allein und zusammen mit $R_2$ die Bedeutung von $R_1$ hat oder, falls n 1 bedeutet, auch Niederalkoxy bedeuten kann und $R_2$, $R_3$, $R_4$, $R_5$ und Ar die im Anspruch 1 angegebene Bedeutung haben, mittels eines Hydrid-Reduktionsmittels die Carbonylgruppe(n) bzw. die gegebenenfalls vorhandene Niederalkoxycarbonylgruppe reduziert, oder

(c) in eine Verbindung der allgemeinen Formel I, in welcher $R_3$ Wasserstoff bedeutet und $R_1$, $R_2$, $R_4$, $R_5$ und Ar die im Anspruch 1 angegebene Bedeutung haben, einen von Wasserstoff verschiedenen Rest $R_3$ einführt, an dessen Bindungskohlenstoffatom sich noch mindestens ein Wasserstoffatom befindet, oder

(d) in eine Verbindung der allgemeinen Formel I, in welcher ein oder beide Symbole $R_1$ und $R_2$ Wasserstoff bedeuten, und $R_3$, $R_4$, $R_5$ und Ar die im Anspruch 1 angegebene Bedeutung haben, einen oder zwei von Wasserstoff verschiedene Reste $R_1$ und/oder $R_2$ einführt,

und gewünschtenfalls eine erhaltene Verbindung der allgemeinen Formel I in ein Additionssalz mit einer anorganischen oder organischen Säure überführt oder aus einem erhaltenen Säureadditionssalz die Base freisetzt und letztere gewünschtenfalls wiederum in ein Säureadditionssalz überführt.

31. Verfahren nach Anspruch 30, dadurch gekennzeichnet, dass man für die Umsetzung gemäss a) als reaktionsfähige Ester von Verbindungen der allgemeinen Formel II Ester organischer Sulfonsäuren oder Halogenide verwendet.

32. Verfahren nach Anspruch 30, dadurch gekennzeichnet, dass man die Umsetzung gemäss a) in Gegenwart eines säurebindenden Mittels durchführt.

33. Verfahren nach Anspruch 30, dadurch gekennzeichnet, dass man die Reduktion gemäss b) mittels eines Niederalkylaluminiumhydrids in einem aromatischen Kohlenwasserstoff, oder mittels Lithiumaluminiumhydrid oder Diboran in einem ätherartigen Lösungsmittel durchführt.

34. Verfahren nach Anspruch 30, dadurch gekennzeichnet, dass man die Einführung eines von Wasserstoff verschiedenen, am Bindungskohlenstoffatom noch mindestens ein Wasserstoffatom aufweisenden Restes $R_3$ in eine Verbindung der allgemeinen Formel I gemäss c) durch Umsetzung einer der letztgenannten Verbindungen mit einem reaktionsfähigen Ester eines primären oder sekundären Niederalkanols, Niederalkenols, eines Cycloalkanols mit 5 bis 7 Kohlenstoffatomen oder eines primären Cycloalkylniederalkanols mit 4 bis 7 Kohlenstoffatomen vollzieht.

35. Verfahren nach Anspruch 34, dadurch gekennzeichnet, dass man als reaktionsfähigen Ester einen Halogenwasserstoffsäureester oder einen Ester mit einer organischen Sulfonsäure verwendet.

36. Verfahren nach Anspruch 34, dadurch gekennzeichnet, dass man die Umsetzung in Gegenwart eines basischen Kondensationsmittels durchführt.

37. Verfahren nach Anspruch 30, dadurch gekennzeichnet, dass man die Einführung von einem bzw. zwei von Wasserstoff verschiedenen Resten $R_1$ und/oder $R_2$ gemäss d) durch Umsetzung einer Verbindung der allgemeinen Formel I, in welcher mindestens eines der Symbole $R_1$ und $R_2$ Wasserstoff bedeutet, mit der äquimolaren oder gegebenenfalls mindestens doppeltmolaren Menge eines reaktionsfähigen Esters eines Niederalkanols oder eines reaktionsfähigen Monoesters eines Niederalkandiols durchführt.

38. Verfahren nach Anspruch 37, dadurch gekennzeichnet, dass man als reaktionsfähigen Ester einen Ester mit einer organischen Sulfonsäure oder einen Halogenwasserstoffsäureester verwendet.

39. Verfahren nach Anspruch 37, dadurch gekennzeichnet, dass man die Umsetzung in Gegenwart eines säurebindenden Mittels durchführt.

40. Verfahren nach Anspruch 30, dadurch gekennzeichnet, dass man die Einführung von einem bzw. zwei von Wasserstoff verschiedenen Resten $R_1$ und/oder $R_2$ gemäss d) durch Umsetzung einer Verbindung der allgemeinen Formel I, in welcher mindestens eines der Symbole $R_1$ und $R_2$ Wasserstoff bedeutet,mit einem Oxoniederalkan unter reduzierenden Bedingungen durchführt.

41. Verfahren nach Anspruch 40, dadurch gekennzeichnet, dass man als Oxoniederalkan Formaldehyd in Gegenwart von Ameisensäure verwendet.

42. Verwendung einer Verbindung der im Anspruch 1 angegebenen allgemeinen Formel I, in welcher $R_1$, $R_2$, $R_3$, $R_4$, $R_5$ und Ar die dort definierte Bedeutung haben, oder eines ihrer pharmazeutisch annehmbaren Säureadditionssalze zur Herstellung von Arzneimitteln auf nicht-chemischem Wege.

43. Verwendung einer Verbindung der im Anspruch 1 angegebenen allgemeinen Formel I, in welcher $R_1$, $R_2$, $R_3$, $R_4$, $R_5$ und Ar die dort definierte Bedeutung haben, oder eines ihrer pharmazeutisch annehmbaren Säureadditionssalze als Antidepressivum und Anxiolyticum.

44. Verwendung einer Verbindung der im Anspruch 1 angegebenen allgemeinen Formel I, in welcher $R_1$, $R_2$, $R_3$, $R_4$, $R_5$ und Ar die dort definierte Bedeutung haben, oder eines ihrer pharmazeutisch annehmbaren Säureadditionssalze zur Behandlung von Depressionen.

45. Behandlung von Depressionen durch Verabreichung einer Verbindung der im Anspruch 1 angegebenen allgemeinen Formel I in welcher $R_1$, $R_2$, $R_3$, $R_4$, $R_5$ und Ar die dort definierte Bedeutung haben, oder eines ihrer pharmazeutisch annehmbaren Säureadditionssalze.

Patentansprüche (für Oesterreich)

1. Verfahren zur Herstellung von neuen polycyclischen Polyazaheterocyclen der allgemeinen Formel I

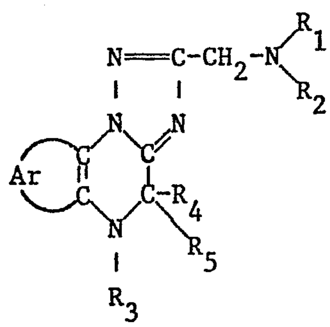

(I)

in welcher $R_1$ und $R_2$ unabhängig voneinander Wasserstoff, Niederalkyl oder Hydroxyniederalkyl, oder zusammen Niederalkylen oder Aethylenoxyäthylen, Aethylenazaäthylen oder N-Niederalkyl- oder N-(2-Hydroxyniederalkyl)-äthylenazaniederäthylen, $R_3$ Wasserstoff oder einen niederen aliphatischen oder gesättigten, niederen cycloaliphatischen Kohlenwasserstoffrest oder unsubstituiertes oder substituiertes Phenyl, $R_4$ und $R_5$ Wasserstoff oder Niederalkyl und Ar einen unsubstituierten oder substituierten Benzo- oder Pyridorest bedeutet, und deren Säureadditionssalzen, dadurch gekennzeichnet, dass man

(a) einen reaktionsfähigen Ester einer Verbindung der allgemeinen Formel II

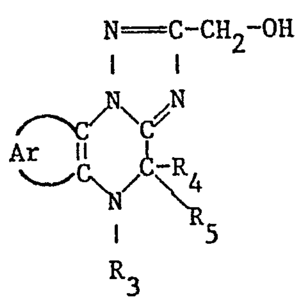

(II) ,

in welcher

$R_3$, $R_4$, $R_5$ und Ar die oben angegebene Bedeutung haben, mit einer Verbindung der allgemeinen Formel III

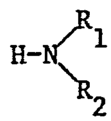

(III) ,

in welcher $R_1$ und $R_2$ die oben angegebene Bedeutung haben, umsetzt, oder

(b) in einer Verbindung der allgemeinen Formel IV

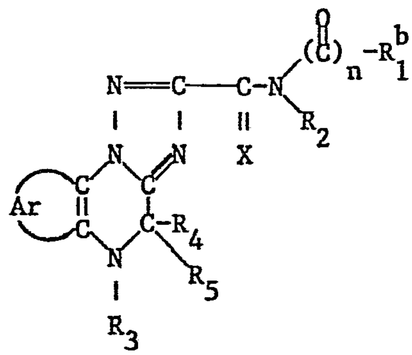

(IV) ,

in welcher n Null oder 1 und X Sauerstoff bedeutet, oder, falls n 1 bedeutet, auch zwei Wasserstoffatome bedeuten kann, $R_1^b$ allein und zusammen mit $R_2$ die Bedeutung von $R_1$ hat oder, falls n 1 bedeutet, auch Niederalkoxy bedeuten kann und $R_2$, $R_3$, $R_4$, $R_5$ und Ar die oben angegebene Bedeutung haben, mittels eines Hydrid-Reduktionsmittels die Carbonylgruppe(n) bzw. die gegebenenfalls vorhandene Niederalkoxycarbonylgruppe reduziert, oder

(c) in eine Verbindung der allgemeinen Formel I, in welcher $R_3$ Wasserstoff bedeutet und $R_1$, $R_2$, $R_4$, $R_5$ und Ar die oben angegebene Bedeutung haben, einen von Wasserstoff verschiedenen Rest $R_3$ einführt, an dessen Bindungskohlenstoffatom sich noch mindestens ein Wasserstoffatom befindet, oder

(d) in eine Verbindung der allgemeinen Formel I, in welcher ein oder beide Symbole $R_1$ und $R_2$ Wasserstoff bedeuten, und $R_3$, $R_4$, $R_5$ und Ar die oben angegebene Bedeutung haben, einen oder zwei von Wasserstoff verschiedene Reste $R_1$ und/oder $R_2$ einführt,

und gewünschtenfalls eine erhaltene Verbindung der allgemeinen Formel I in ein Additionssalz mit einer anorganischen oder organischen Säure überführt oder aus einem erhaltenen Säureadditionssalz die Base freisetzt und letztere gewünschtenfalls wiederum in ein Säureadditionssalz überführt.

2. Verfahren nach Anspruch 1, dadurch gekennzeichnet, dass man für die Umsetzung gemäss a) als reaktionsfähige Ester von Verbindungen der allgemeinen Formel II Ester organischer Sulfonsäuren oder Halogenide verwendet.

3. Verfahren nach Anspruch 1, dadurch gekennzeichnet, dass man die Umsetzung gemäss a) in Gegenwart eines säurebindenden Mittels durchführt.

4. Verfahren nach Anspruch 1, dadurch gekennzeichnet, dass man die Reduktion gemäss b) mittels eines Niederalkylaluminiumhydrids in einem aromatischen Kohlenwasserstoff, oder mittels Lithiumaluminiumhydrid oder Diboran in einem ätherartigen Lösungsmittel durchführt.

5. Verfahren nach Anspruch 1, dadurch gekennzeichnet, dass man die Einführung eines von Wasserstoff verschiedenen, am Bindungskohlenstoffatom noch mindestens ein Wasserstoffatom aufweisenden Restes $R_3$ in eine Verbindung der allgemeinen Formel I gemäss c) durch Umsetzung einer der letztgenannten Verbindungen mit einem reaktionsfähigen Ester eines primären oder sekundären Niederalkanols, Niederalkenols, eines Cycloalkanols mit 5 bis 7 Kohlenstoffatomen oder eines primären Cycloalkylniederalkanols mit 4 bis 7 Kohlenstoffatomen vollzieht.

6. Verfahren nach Anspruch 5, dadurch gekennzeichnet, dass man als reaktionsfähigen Ester einen Halogenwasserstoffsäureester oder einen Ester mit einer organischen Sulfonsäure verwendet.

7. Verfahren nach Anspruch 5, dadurch gekennzeichnet, dass man die Umsetzung in Gegenwart eines basischen Kondensationsmittels durchführt.

8. Verfahren nach Anspruch 1, dadurch gekennzeichnet, dass man die Einführung von einem bzw. zwei von Wasserstoff verschiedenen Resten $R_1$ und/oder $R_2$ gemäss d) durch Umsetzung einer Verbindung der allgemeinen Formel I, in welcher mindestens eines der Symbole $R_1$ und $R_2$ Wasserstoff bedeutet, mit der äquimolaren oder gegebenenfalls mindestens doppeltmolaren Menge eines reaktionsfähigen Esters eines Niederalkanols oder eines reaktionsfähigen Monoesters eines Niederalkandiols durchführt.

9. Verfahren nach Anspruch 8, dadurch gekennzeichnet, dass man als reaktionsfähigen Ester einen Ester mit einer organischen Sulfonsäure oder einen Halogenwasserstoffsäureester verwendet.

10. Verfahren nach Anspruch 8, dadurch gekennzeichnet, dass man die Umsetzung in Gegenwart eines säurebindenden Mittels durchführt.

11. Verfahren nach Anspruch 1, dadurch gekennzeichnet, dass man die Einführung von einem bzw. zwei von Wasserstoff verschiedenen Resten $R_1$ und/oder $R_2$ gemäss d) durch Umsetzung einer Verbindung der allgemeinen Formel I, in welcher mindestens eines der Symbole $R_1$ und $R_2$ Wasserstoff bedeutet, mit einem Oxoniederalkan unter reduzierenden Bedingungen durchführt.

12. Verfahren nach Anspruch 11, dadurch gekennzeichnet, dass man als Oxoniederalkan Formaldehyd in Gegenwart von Ameisensäure verwendet.

13. Verfahren nach Anspruch 1, dadurch gekennzeichnet, dass man eine Verbindung der im Anspruch 1 angegebenen allgemeinen Formel I, in der $R_1$ und $R_2$ die im Anspruch 1 angegebene Bedeutung haben, $R_3$ einen niederen aliphatischen oder einen gesättigten niederen cycloaliphatischen Kohlenwasserstoffrest oder unsubstituiertes oder durch Halogen bis Atomnummer 35, Niederalkyl, Niederalkoxy, Niederalkylthio, Trifluormethyl, Cyano oder Nitro substituiertes Phenyl und Ar einen unsubstituierten oder durch Substituenten der vorgenannten Art substituierten Benzo- oder Pyrido[2,3-e]rest bedeutet und $R_4$ und $R_5$ die im Anspruch 1 angegebene Bedeutung haben, und deren Säureadditionssalze herstellt.

14. Verfahren nach Anspruch 1, dadurch gekennzeichnet, dass man eine Verbindung der im Anspruch 1 angegebenen allgemeinen Formel I, in der $R_1$ Niederalkyl, $R_2$ Wasserstoff oder Niederalkyl, oder $R_1$ und $R_2$ zusammen Niederalkylen, $R_3$ unsubstituiertes oder durch Halogen bis Atomnummer 35 substituiertes Phenyl oder niederes Cycloalkyl und Ar einen unsubstituierten oder durch Halogen bis Atomnummer 35 substituierten Benzo- oder Pyrido[2,3-e]rest bedeutet und $R_4$ und $R_5$ die im Anspruch 1 angegebene Bedeutung haben, und deren Säureadditionssalze herstellt.

15. Verfahren nach Anspruch 1, dadurch gekennzeichnet, dass man eine Verbindung der im Anspruch 1 angegebenen allgemeinen Formel I, in der $R_1$ Niederalkyl, $R_2$ Wasserstoff oder Niederalkyl oder $R_1$ und $R_2$ Niederalkylen, $R_3$ unsubstituiertes oder durch Halogen bis Atomnummer 35 substituiertes Phenyl, und Ar einen unsubstituierten oder durch Halogen bis Atomnummer 35 substituierten Benzorest oder den Pyrido[2,3-e]rest bedeutet, $R_4$ die im Anspruch 1 angegebene Bedeutung hat und $R_5$ Wasserstoff oder Methyl bedeutet, und deren Säureadditionssalze herstellt.

16. Verfahren nach Anspruch 1, dadurch gekennzeichnet, dass man eine Verbindung der im Anspruch 1 angegebenen allgemeinen Formel I, in der $R_1$ Methyl, $R_2$ Wasserstoff oder $R_1$ und $R_2$ Tetramethylen, $R_3$

unsubstituiertes oder durch Chlor substituiertes Phenyl, und Ar einen unsubstituierten oder durch Chlor substituierten Benzorest oder den Pyrido[2,3-e]rest bedeutet, $R_4$ Wasserstoff oder Methyl und $R_5$ Wasserstoff oder Methyl bedeutet, und deren Säureadditionssalze herstellt.

17. Verfahren nach Anspruch 1, dadurch gekennzeichnet, dass man eine Verbindung der im Anspruch 1 angegebenen allgemeinen Formel I, in der $R_1$ Methyl, $R_2$ Wasserstoff oder Methyl, $R_3$ Phenyl, $R_4$ Wasserstoff oder Methyl, $R_5$ Wasserstoff oder Methyl und Ar den Benzo- oder Pyrido[2,3-e]rest bedeutet, und deren Säureadditonssalze herstellt.

18. Verfahren nach Anspruch 1, dadurch gekennzeichnet, dass man 2-[(Dimethylamino)-methyl]-5-phenyl-4,5-dihydro-[1,2,4]-triazolo-[1,5-a]chinoxalin und dessen Säureadditionssalze herstellt.

0074929

Nummer der Anmeldung

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

EP 82 81 0381

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | Keine Entgegenhaltungen | | C 07 D 487/04<br>471/14<br>A 61 K 31/495<br>(C 07 D 487/04<br>249/00<br>241/00)<br>(C 07 D 471/14<br>249/00<br>241/00<br>221/00) |
| | | | **RECHERCHIERTE SACHGEBIETE (Int. Cl. 3)** |
| | | | C 07 D 487/00<br>471/00<br>A 61 K 31/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-44

Unvollständig recherchierte Patentansprüche:

Nicht recherchierte Patentansprüche: 45

Grund für die Beschränkung der Recherche: Verfahren zur chirurgischen oder therapeutischen Behandlung des menschlichen oder tierischen Körpers (siehe Art. 52(4) des Europäischen Patentübereinkommens)

| Recherchenort | Abschlußdatum der Recherche | Prufer |
|---|---|---|
| Den Haag | 20-12-1942 | ALFARO |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument